# EUROPÄISCHE PATENTSCHRIFT

(11) **EP 4 486 233 B1**
(45) Veröffentlichungstag und Bekanntmachung des Hinweises auf die Patenterteilung: **14.01.2026**
(21) Anmeldenummer: 23703773.4
(22) Anmeldetag: 06.02.2023
(51) Int. Cl.: A61B 17/68, A61B 17/80

(54) **IMPLANTAT ZUR FIXIERUNG EINES KRANIALEN KNOCHENDECKELS IN EINER KRANIALEN ÖFFNUNG**
IMPLANT FOR FIXING A CRANIAL BONE FLAP IN A CRANIAL OPENING
IMPLANT POUR FIXER UN VOLET CRÂNIEN DANS UNE OUVERTURE CRÂNIENNE

(30) Priorität: 28.02.2022 DE 102022202037
(43) Veröffentlichungstag der Anmeldung: 08.01.2025
(73) Patentinhaber: Aesculap AG, 78532 Tuttlingen (DE)
(72) Erfinder: HAGEN, Thomas, 78532 Tuttlingen (DE); KUEMMERLIN, Jana, 93055 Regensburg (DE); DROST, Erick, 78727 Oberndorf a.N. (DE); ONKEN, Jakob, 55116 Mainz (DE); NONNENMANN, Martin, 78573 Wurmlingen (DE); SCHWARZ, Tina, 78628 Rottweil (DE); PLEIL, Thomas, 78073 Bad Dürrheim (DE)
(74) Vertreter: Patentanwälte Ruff, Wilhelm, Beier, Dauster & Partner mbB
(86) Internationale Anmeldenummer: PCT/EP2023/052850
(87) Internationale Veröffentlichungsnummer: WO 2023/160999

(56) Entgegenhaltungen:
- DE-A1- 102004 054 122
- US-A1- 2015 150 612
- US-B1- 6 197 030

## Beschreibung

Die Erfindung betrifft ein Implantat zur Fixierung eines kranialen Knochendeckels in einer kranialen Öffnung.

Neurochirurgische Eingriffe am Gehirn erfolgen in der Regel durch eine Öffnung des knöchernen Schädels. Das Herstellen einer solchen kranialen Öffnung wird auch als Kraniotomie bezeichnet. Bei einer Kraniotomie wird ein Teil des Schädelknochens beispielsweise durch Trepanation aus dem knöchernen Schädel herausgetrennt. Das herausgetrennte Teil wird auch als Knochendeckel oder -fragment bezeichnet. Nach erfolgtem Eingriff wird der Knochendeckel in der zuvor hergestellten kranialen Öffnung repositioniert und fixiert.

Zur Fixierung des Knochendeckels sind Implantate in Form sogenannter Klammersysteme bekannt. Beispielsweise ist ein Klammersystem der Aesculap AG, Tuttlingen, Deutschland, unter der Bezeichnung CranioFix^{®}2 bekannt. Das bekannte Klammersystem weist ein oberes Tellerelement, ein unteres Tellerelement sowie ein die beiden Tellerelemente beweglich miteinander verbindendes Stabelement auf. Das Stabelement ist entlang einer Längsachse zwischen einem oberen Ende und einem unteren Ende längserstreckt, wobei das obere Tellerelement an dem oberen Ende und das untere Tellerelement an dem unteren Ende angebracht sind. Zur Fixierung des Knochendeckels wird zunächst das untere Tellerelement im epiduralen Raum platziert, wobei das Stabelement in etwa parallel zur Axialrichtung der kranialen Öffnung ausgerichtet wird. Der Knochendeckel wird in der kranialen Öffnung repositioniert, wobei dessen Innenseite auf das untere Tellerelement aufgelegt wird. Das Stabelement ist hierbei ausgehend von der Schädelinnenseite durch den zwischen dem Knochendeckel und dem umgebenden Schädelknochen ausgebildeten Ringspalt auf die Schädelaußenseite erstreckt. Zur endgültigen Fixierung wird das obere Tellerelement entlang des Stabelements - und damit in Axialrichtung der kranialen Öffnung - an das untere Tellerelement angenähert. Hierfür sind Schraub- oder Rastmechanismen etabliert. Im Endzustand der Fixierung sind die beiden Tellerelemente in Axialrichtung der kranialen Öffnung einerseits fest auf die Außenseite und andererseits fest auf die Innenseite des Knochendeckels und des umgebenden Schädelknochens gepresst.

Im Hinblick auf das bekannte Klammersystem können unterschiedliche Aspekte als nachteilig erachtet werden: Die Anordnung des unteren Tellerelements im epiduralen Raum kann unter gewissen Voraussetzungen zu einer Reizung der Dura mater und schlechtestenfalls zu einem Epiduralhämatom führen. Denn das axiale Verspannen der Tellerelemente birgt das Risiko, dass ein unbeabsichtigter Druck auf die Dura mater aufgebracht wird, was letztlich zu dem besagten Epiduralhämatom führen kann. Um eine unbeabsichtigte Druckkraftbeaufschlagung der Dura mater zu vermeiden, ist im Falle von CranioFix^{®}2 ein gesondertes Werkzeug zum Halten des Stabelements vorgesehen. Das zusätzlich notwendige Werkzeug kann zu einer insgesamt erschwerten Handhabung führen. Überdies verbleibt das obere Tellerelement nach erfolgter Fixierung dauerhaft an der Schädelaußenseite. Dies ist insbesondere in kosmetisch kritischen Bereichen, beispielsweise bei einer frontalen Kraniotomie, nachteilig. Da das obere und das untere Tellerelement zur zufriedenstellenden Fixierung möglichst plan auf dem Schädelknochen und dem Knochendeckel aufliegen müssen, ist eine Fixierung bei starken Schädelkrümmungen oder Dickegradienten oftmals nicht oder nicht zufriedenstellend möglich. Im Übrigen können starke Krümmungen dazu führen, dass das obere Tellerelement von der Schädelaußenseite absteht, was zu unzureichenden kosmetischen Ergebnissen oder auch Kopfhautreizungen führen kann.

Ein Implantat mit den Merkmalen des Oberbegriffs des Anspruchs 1 ist aus der US 2015/150612 A1 bekannt.

Weitere gattunsgemäße Implantate sind aus DE 10 2004 054122 A1 und US 6 197 030 B1 bekannt.

Aufgabe der Erfindung ist es, ein Implantat der eingangs genannten Art bereitzustellen, welches Vorteile gegenüber dem Stand der Technik bietet. Insbesondere sollen die mit dem Stand der Technik einhergehenden Nachteile wenigstens teilweise überwunden oder vermindert werden.

Diese Aufgabe wird durch das Bereitstellen eines Implantats mit den Merkmalen des Anspruchs 1 gelöst. Vorteilhafte Ausgestaltungen sind Gegenstand der Unteransprüche. Deren Wortlaut wird durch ausdrückliche Bezugnahme zum Inhalt der Beschreibung gemacht.

Das erfindungsgemäße Implantat zur Fixierung eines kranialen Knochendeckels in einer kranialen Öffnung weist auf: eine Längsachse, welche entlang einer Axialrichtung der kranialen Öffnung ausgerichtet ist, eine Querachse, welche entlang einer Radialrichtung der kranialen Öffnung ausgerichtet ist, einen ersten Abschnitt, welcher zur Kraftübertragung auf den Knochendeckel eingerichtet ist, einen zweiten Abschnitt, welcher zur Kraftübertragung auf einen die kraniale Öffnung umrandenden Schädelknochen eingerichtet ist, wobei der erste Abschnitt und/oder der zweite Abschnitt zur Anordnung in einem zwischen einem Außenumfang des Knochendeckels und einem Innenumfang des Schädelknochens ausgebildeten Ringspalt eingerichtet ist, und einen mit dem ersten Abschnitt und dem zweiten Abschnitt wirkverbundenen Mechanismus, mittels dessen ein auf die Querachse projizierter Querabstand zwischen dem ersten Abschnitt und dem zweiten Abschnitt wenigstens vergrößerbar ist, wodurch der erste Abschnitt in Radialrichtung nach innen an den Knochendeckel und der zweite Abschnitt in Radialrichtung nach außen an den Schädelknochen anpressbar sind. Die erfindungsgemäße Lösung erlaubt eine - in Bezug auf die Orientierung der kranialen Öffnung - radiale Fixierung. Die zur Fixierung mittels des Implantats auf den Knochendeckel einerseits und den umgebenden Schädelknochen andererseits aufgebrachten Kräfte wirken folglich radial. Mit anderen Worten ausgedrückt, wirken die aufgebrachten Kräfte in der Ebene des Schädelknochens bzw. des Knochendeckels und nicht etwa in deren Dickenrichtung. Dies im Unterschied zu aus dem Stand der Technik bekannten Lösungen, welche eine axiale Fixierung vorsehen. Die radiale Fixierung vermeidet zum einen eine unbeabsichtigte Druckkraftbeaufschlagung der Dura mater und die hiermit einhergehenden Risiken. Zum wird davon ausgegangen, dass eine radiale, d.h. in der Ebene wirkende, Kraftbeaufschlagung zu einer verbesserten Regeneration des knöchernen Schädels führen kann. Die zur Fixierung notwendige Kraftbeaufschlagung erfolgt bei der erfindungsgemäßen Lösung über eine Veränderung, genauer: Vergrößerung, des auf die Querachse projizierten Querabstands zwischen dem ersten und dem zweiten Abschnitt. Da die Querachse entlang, bevorzugt parallel, zu der Radialrichtung der kranialen Öffnung ausgerichtet ist, bewirkt die Vergrößerung des projizierten Querabstands das besagte radiale Anpressen an den Knochendeckel einerseits und den Schädelknochen andererseits. Hierdurch wird der Ringspalt im Bereich des Implantats vergrößert, was zwangsläufig zu einer Verringerung des Ringspalts auf einer dem Implantat diametral gegenüberliegenden Seite der kranialen Öffnung führt. An der besagten diametral gegenüberliegenden Seite werden der Knochendeckel und der umrandende Schädelknochen folglich unter Einwirkung des Implantats aneinandergepresst und letztlich fixiert. Der hierbei auftretende Knochen-Knochen-Kontakt ist der verbesserten Regeneration zuträglich. Insbesondere für den Fall, dass mehrere in Umfangsrichtung des Knochendeckels beabstandete Implantate verwendet werden, ist ein solcher Knochen-Knochen-Kontakt nicht unbedingt notwendig. Der mit dem ersten Abschnitt und dem zweiten Abschnitt wirkverbundene Mechanismus ist wenigstens zur Vergrößerung des projizierten Querabstands zwischen den besagten Abschnitten eingerichtet. Die Vergrößerung des Querabstands kann auch als Aufspreizen oder Aufspreizbewegung bezeichnet werden. Unterschiedliche Ausgestaltungen der Erfindung weisen zum Generieren der Aufspreizbewegung unterschiedliche Mechanismen auf. Beispielsweise kann die Aufspreizbewegung durch eine translatorische und/oder rotatorische Bewegung, insbesondere Relativbewegung, der beiden Abschnitte bewirkt sein. Zur Kraftübertragung auf den Knochendeckel ist der erste Abschnitt bei unterschiedlichen Ausgestaltungen der Erfindung unterschiedlich ausgebildet. Bei einer Ausgestaltung wirkt der erste Abschnitt, vorzugsweise unmittelbar, auf einen Außenumfang des Knochendeckels. Bei einer weiteren Ausgestaltung ist der erste Abschnitt in eine radial in den Außenumfang des Knochendeckels erstreckte Bohrung eingefügt. Entsprechendes gilt mutatis mutandis hinsichtlich des zweiten Abschnitts. Der Mechanismus ist vorzugsweise zur manuellen Betätigung durch den Operateur eingerichtet. Die Betätigung kann rein manuell oder unter Zuhilfenahme eines hierfür geeigneten Werkzeugs erfolgen. Die Längsachse und die Querachse des Implantats sind orthogonal zueinander ausgerichtet. Zur Fixierung und/oder in einem fixierten Zustand des kranialen Knochendeckels ist die Längsachse entlang, vorzugsweise parallel zu, der Axialrichtung der kranialen Öffnung ausgerichtet. Die Querachse ist hierbei entlang, vorzugsweise parallel zu, der Radialrichtung der kranialen Öffnung orientiert. Bei bevorzugten Ausgestaltungen ist der Mechanismus zusätzlich zur Verringerung des projizierten Querabstands eingerichtet. Eine Verringerung des projizierten Querabstands erlaubt ein einfaches Lösen einer zuvor vorgenommenen Fixierung. Dies vereinfacht Revisionseingriffe.

In Ausgestaltung der Erfindung ist der Mechanismus zur translatorischen Verlagerung des ersten Abschnitts und/oder des zweiten Abschnitts entlang der Querachse eingerichtet. Zum Generieren der besagten Aufspreizbewegung wird folglich wenigstens einer der beiden Abschnitte translatorisch entlang der Querachse verlagert. Bei einer Ausgestaltung gestattet der Mechanismus eine Verlagerung des ersten Abschnitts entlang der Querachse, wobei der zweite Abschnitt relativ zu der Querachse festgelegt ist. Bei einer weiteren Ausgestaltung gestattet der Mechanismus eine Verlagerung des zweiten Abschnitts entlang der Querachse, wobei der erste Abschnitt relativ zu der Querachse festgelegt ist. Bei einer weiteren Ausgestaltung gestattet der Mechanismus eine Verlagerung beider Abschnitte entlang der Querachse, wobei die Abschnitte hierbei in entgegengesetzte Richtungen verlagert werden.

In weiterer Ausgestaltung der Erfindung ist der Mechanismus zur rotatorischen Verlagerung des ersten Abschnitts und/oder des zweiten Abschnitts um die Längsachse eingerichtet. Um die Aufspreizbewegung zu generieren, wird bei dieser Ausgestaltung wenigstens einer der beiden Abschnitte rotatorisch um die Längsachse verlagert. Bei einer Ausgestaltung gestattet der Mechanismus eine rotatorische Verlagerung des ersten Abschnitts, wobei der zweite Abschnitt relativ zu der Längsachse und/oder Querachse festgelegt ist. Bei einer weiteren Ausgestaltung gestattet der Mechanismus eine rotatorische Verlagerung des zweiten Abschnitts, wobei der erste Abschnitt relativ zu der Längsachse und/oder Querachse festgelegt ist. Bei einer weiteren Ausgestaltung der Erfindung bewirkt der Mechanismus eine, vorzugsweise gleichsinnige, rotatorische Verlagerung beider Abschnitte um die Längsachse.

In weiterer Ausgestaltung der Erfindung weist der erste Abschnitt eine erste Kontaktfläche auf, welche zur radialen Anlage an dem Außenumfang des Knochendeckels eingerichtet ist. Alternativ oder zusätzlich weist der zweite Abschnitt eine zweite Kontaktfläche auf, welche zur radialen Anlage an dem Innenumfang des Schädelknochens eingerichtet ist. Bei dieser Ausgestaltung der Erfindung erfolgt die Kraftübertragung folglich über eine, vorzugsweise unmittelbare, Kontaktpaarung zwischen dem betreffenden Abschnitt und der jeweiligen Umfangsfläche der zugeordneten Knochenstruktur, d.h. dem Knochendeckel oder dem umgebenden Schädelknochen. Hierdurch kann insbesondere auf eine spezielle Vorbereitung oder Bearbeitung der jeweiligen Knochenstruktur, insbesondere das Einbringen von Bohrungen oder dergleichen, verzichtet werden. Der Knochendeckel weist üblicherweise eine in etwa runde Form auf. Entsprechendes gilt sinngemäß hinsichtlich der Form der kranialen Öffnung. Folglich ist eine Wölbung des Außenumfangs des Knochendeckels üblicherweise konvex. Die Wölbung des die kraniale Öffnung berandenden Schädelknochens ist folglich konkav. Vorzugsweise ist die erste Kontaktfläche und/oder die zweite Kontaktfläche dementsprechend komplementär gewölbt. Hierdurch kann eine möglichst vollflächige Anlage der ersten Kontaktfläche an dem Außenumfang des Knochendeckels und/oder der zweiten Kontaktfläche an dem Innenumfang des Schädelknochens erreicht werden.

In weiterer Ausgestaltung der Erfindung weist der erste Abschnitt eine Fügefläche auf, welche zum Einfügen in eine radial in den Außenumfang des Knochendeckels erstreckte Bohrung eingerichtet ist. Die besagte Bohrung kann in einem gesonderten Operationsschritt radial in den Knochendeckel gebohrt werden und dient einer wenigstens abschnittsweisen Aufnahme des ersten Abschnitts. Der erste Abschnitt ist komplementär zu der Bohrung zylindrisch gestaltet und weist vorzugsweise einen kreisrunden Querschnitt auf. Die Fügefläche umfasst vorzugsweise wenigstens eine Außenmantelfläche und/oder eine Stirnendfläche des ersten Abschnitts. Zwecks Positionierung und/oder Vorfixierung wird der erste Abschnitt in Axialrichtung der Bohrung in dieselbe eingesteckt. Der hierbei etablierte Form- und/oder Kraftschluss verhindert ein ungewolltes Abgleiten des ersten Abschnitts an dem Außenumfang des Knochendeckels. Wichtig zu erkennen ist, dass das Einfügen des ersten Abschnitts, genauer: dessen Fügefläche, in die Bohrung für sich allein genommen noch nicht die Fixierung etabliert. Die Fixierung erfolgt nach dem Einfügen des ersten Abschnitts in die Bohrung und über das Aufbringen der besagten Aufspreizbewegung, d.h. der Vergrößerung des projizierten Querabstands zwischen dem ersten Abschnitt und dem zweiten Abschnitt.

Erfindungsgemäß ist ein Stützabschnitt vorhanden, welcher entlang der Längsachse oberhalb des ersten Abschnitts und des zweiten Abschnitts angeordnet ist und eine Unterseite aufweist, welche zum Abstützen auf einer Außenseite des Schädelknochens und einer Außenseite des Knochendeckels eingerichtet ist. Der Stützabschnitt dient einer vereinfachten Positionierung des Implantats bei der Fixierung und verhindert eine ungewollte axiale Verlagerung in Richtung des Epiduralraums. Die Unterseite des Stützabschnitts ist der Außenseite des knöchernen Schädels zugewandt und/oder entlang der Längsachse orientiert. Bei unterschiedlichen Ausgestaltungen weist der Stützabschnitt unterschiedliche Gestaltgebungen auf, wobei insbesondere eine Platten-, Scheiben- und/oder Ringform denkbar sind. In abgestütztem Zustand liegt die Unterseite auf einer Seite des Ringspalts auf der Außenseite des Knochendeckels und auf der gegenüberliegenden Seite des Ringspalts auf der Außenseite des Schädelknochens auf. Der Stützabschnitt ist entlang der Querachse zwischen dem Knochendeckel einerseits und dem Schädelknochen andererseits längserstreckt.

Erfindungsgemäß ist der Stützabschnitt mittels einer lösbaren Fügeverbindung, insbesondere einem Bajonettverschluss, mit dem Implantat im Übrigen verbunden. Diese Ausgestaltung erlaubt ein Entfernen des Stützabschnitts nach erfolgter Fixierung. Zu diesem Zweck ist die besagte lösbare Fügeverbindung zwischen dem Stützabschnitt und wenigstens einem weiteren Bauteil und/oder Abschnitt des Implantats ausgebildet. Vorzugsweise ist die lösbare Fügeverbindung ein Bajonettverschluss. Durch das Entfernen des Stützabschnitts nach der erfolgten Fixierung kann die Bauhöhe des Implantats in Bezug auf die Längsachse reduziert werden. Insbesondere wird vermieden, dass der Stützabschnitt permanent an der Außenseite des knöchernen Schädels verbleibt und hierdurch zu einer kosmetischen, medizinischen und/oder anderweitigen Beeinträchtigung führt.

In weiterer Ausgestaltung der Erfindung ist wenigstens ein Dornabschnitt vorhanden, welcher entlang der Querachse längserstreckt ist und einends eine über den ersten Abschnitt hinausragende Dornspitze aufweist, welche zum radialen Einstechen in den Außenumfang des Knochendeckels eingerichtet ist. Der Dornabschnitt dient einer Vorfixierung zwischen Knochendeckel und Implantat. Zur Vorfixierung werden der Knochendeckel und das Implantat mittels des Dornabschnitts - noch abseits der kranialen Öffnung - manuell zusammengesteckt. Zu diesem Zweck ist die Dornspitze zum radialen Einstechen in den Knochendeckel eingerichtet. In einem solchen vorfixierten Zustand können der Knochendeckel und das Implantat gemeinsam in der kranialen Öffnung repositioniert werden. Erst hiernach erfolgt die eigentliche Fixierung. Um ein anforderungsgerechtes Einstechen in den Außenumfang des Knochendeckels zu ermöglichen, ragt die Dornspitze entlang der Querachse über den ersten Abschnitt. Bei unterschiedlichen Ausgestaltungen ist eine unterschiedliche Anzahl von Dornabschnitten vorhanden. Mehrere Dornabschnitte sind insbesondere dann vorteilhaft, wenn der zu fixierende Knochendeckel aus mehreren losen Knochenfragmenten gebildet ist. Diese losen Knochenfragmente können mittels der besagten mehreren Dornabschnitte gemeinsam an dem Implantat vorfixiert werden.

In weiterer Ausgestaltung der Erfindung weist der Mechanismus wenigstens ein Spannelement und ein Kegelelement auf, wobei das Spannelement entlang der Querachse beweglich gelagert ist und eine Innenkonusfläche sowie eine den ersten Abschnitt oder den zweiten Abschnitt bildende Stirnfläche aufweist, wobei das Kegelelement entlang der Längsachse beweglich gelagert ist und eine mit der Innenkonusfläche zusammenwirkende Außenkonusfläche aufweist, und wobei das Spannelement mittels einer Bewegung des Kegelelements entlang der Querachse verlagerbar ist. Das Spannelement dient der Kraftübertragung auf den Knochendeckel oder den umrandenden Schädelknochen, je nachdem, ob die Stirnfläche den ersten oder den zweiten Abschnitt bildet. Die Verlagerung des Spannelements entlang der Querachse wird durch eine Längsbewegung des Kegelelements hervorgerufen. Die Innenkonusfläche und die Außenkonusfläche sind in Bezug auf die Längs- und/oder Querachse geneigt und liegen gleitbeweglich aneinander an. Hierdurch bewirkt eine Längsverlagerung des Kegelelements eine Querverlagerung des Spannelements. Das Kegelelement ist vorzugsweise zur direkten oder indirekten manuellen Betätigung eingerichtet. Folglich kann eine zur Verlagerung des Kegelelements entlang der Längsachse erforderliche Kraft- oder Drehmomentbeaufschlagung manuell oder unter Zuhilfenahme eines hierfür geeigneten Werkzeugs direkt oder indirekt auf das Kegelelement aufgebracht werden.

In weiterer Ausgestaltung der Erfindung weist der Mechanismus ein weiteres Spannelement auf, welches mittels der Bewegung des Kegelelements entlang der Querachse entgegengesetzt verlagerbar ist. Hinsichtlich der grundsätzlichen Funktion und Gestaltung des weiteren Spannelements gilt mutatis mutandis das zu dem Spannelement Gesagte. Sofern die Stirnfläche des Spannelements den ersten Abschnitt bildet, bildet eine Stirnfläche des weiteren Spannelements den zweiten Abschnitt oder umgekehrt. Vorzugsweise sind das Spannelement und das weitere Spannelement in Umfangsrichtung des Implantats um 180° zueinander versetzt angeordnet.

In weiterer Ausgestaltung der Erfindung weist das Kegelelement ein Gewinde auf, welches entlang der Längsachse gewindebeweglich mit einem komplementären Gegengewinde verschraubt ist. Zur Kraft- und Bewegungsübertragung auf das Spannelement ist das Kegelelement folglich entlang der Längsachse schraubbeweglich gelagert. Vorzugsweise ist das Gewinde ein Innengewinde und das komplementäre Gegengewinde ist ein Außengewinde. Das komplementäre Gegengewinde ist in Bezug auf die Längsachse feststehend. Infolge der gewindebeweglichen Lagerung bewirkt eine um die Längsachse gerichtete Drehmomentbeaufschlagung eine rotatorische Verlagerung um und eine translatorische Verlagerung entlang der Längsachse. Mit anderen Worten ausgedrückt, kann das Kegelelement bei dieser Ausgestaltung entlang der Längsachse in Richtung der Schädelinnenseite herabgeschraubt und/oder in Richtung der Schädelaußenseite hinaufgeschraubt werden. Bei einer alternativen Ausgestaltung ist anstelle einer gewindebeweglichen Lagerung eine rastbewegliche Lagerung des Kegelelements vorgesehen.

In weiterer Ausgestaltung der Erfindung weist der Mechanismus eine entlang der Längsachse orientierte Drehachse und ein um die Drehachse beweglich gelagertes Exzenterelement mit einer in Bezug auf die Drehachse exzentrischen Kontur auf, wobei unterschiedliche Abschnitte der Kontur den ersten Abschnitt und den zweiten Abschnitt bilden, und wobei eine Drehung des Exzenterelements ein Verspannen der Kontur zwischen dem Außenumfang des Knochendeckels und dem Innenumfang des Schädelknochens bewirkt. Vorzugsweise sind der erste Abschnitt und der zweite Abschnitt in Bezug auf die Drehachse diametral gegenüberliegend an dem Exzenterelement angeordnet. Die exzentrische Kontur ist bei unterschiedlichen Ausgestaltungen unterschiedlich geformt und beispielsweise im weitesten Sinne unrund, oval, länglich oder dergleichen. Die Drehachse ist vorzugsweise parallel zu der Längsachse orientiert. Durch eine Rotation des Exzenterelements um die Drehachse gelangen der erste und der zweite Abschnitt an der jeweiligen Knochenstruktur zur Anlage. Eine weitere Drehung des Exzenterelements bewirkt - aufgrund der exzentrischen Kontur - ein radiales Anpressen des ersten Abschnitts an den Knochendeckel einerseits und andererseits des zweiten Abschnitts an den umrandenden Schädelknochen. Hierdurch wird das Exzenterelement innerhalb des Ringspalts verspannt, der radiale Abstand zwischen dem Außenumfang des Knochendeckels und dem Innenumfang des Schädelknochens wird im Bereich des Exzenterelements vergrößert, folglich wird ein dem Implantat diametral gegenüberliegender Bereich des Ringspalts verkleinert und schließlich unter Ausbildung eines Knochen-Knochen-Kontakts zwischen dem Knochendeckel und dem umrandenden Schädelknochen vollständig geschlossen.

In weiterer Ausgestaltung der Erfindung ist ein Schaftelement vorhanden, an welchem einends das Exzenterelement und andernends ein Werkzeugaufnahmeelement angeordnet sind, wobei das Werkzeugaufnahmeelement zum Aufbringen eines um die Drehachse gerichteten Drehmoments eingerichtet ist. Der Schaft ist entlang, vorzugsweise parallel, zu der Längsachse längserstreckt. Die Werkzeugaufnahme ist an einem ersten Ende und das Exzenterelement ist an einem zweiten Ende des Schaftelements angeordnet. Das Exzenterelement und das Werkzeugaufnahmeelement sind mittels des Schaftelements fest miteinander verbunden, so dass eine Drehung des Werkzeugaufnahmeelements eine dementsprechende Drehung des Schaft- und des Exzenterelements bewirkt. Das Werkzeugaufnahmeelement ist bei unterschiedlichen Ausgestaltungen unterschiedlich ausgeführt und zur Aufnahme eines Werkzeugs eingerichtet. Beispielsweise kann das Werkzeugaufnahmeelement eine Innensechskant-, Außensechskant-, Kreuzschlitz- oder Schlitzaufnahme aufweisen.

In weiterer Ausgestaltung der Erfindung bildet das Werkzeugaufnahmeelement ein in Bezug auf die Längsachse oberes Stirnende des Implantats und ist über einen Sollbruchabschnitt mit dem Schaftelement verbunden. Der Sollbruchabschnitt bricht unter Einwirkung einer definierten mechanischen Belastung. Hierdurch kann das Werkzeugaufnahmeelement nach erfolgter Fixierung auf definierte Weise von dem Schaftelement abgeschert und/oder abgebrochen werden. Da das Werkzeugaufnahmeelement bei dieser Ausgestaltung das obere Stirnende des Implantats bildet, wird hierdurch die Bauhöhe des Implantats in Bezug auf die Längsachse verringert. Insbesondere wird vermieden, dass das Werkzeugaufnahmeelement nach erfolgter Fixierung permanent auf der Außenseite des knöchernen Schädels verbleibt und hierdurch zu kosmetischen, medizinischen und/oder anderweitigen Beeinträchtigungen führt. Der Sollbruchabschnitt ist in Bezug auf die Längsachse zwischen dem Werkzeugaufnahmeelement und dem Schaftelement angeordnet. Im Vergleich zu dem Schaftelement weist der Sollbruchabschnitt einen kleineren lasttragenden Querschnitt auf. Ein- und dieselbe mechanische Belastung führt hierdurch folglich zu einer im Bereich des Sollbruchabschnitts lokal höheren mechanischen Beanspruchung und letztlich zu einem lokalen mechanischen Versagen.

In weiterer Ausgestaltung der Erfindung weist das Exzenterelement wenigstens einen ersten Klingenabschnitt, welcher den ersten Abschnitt bildet, und einen um die Drehachse versetzt angeordneten zweiten Klingenabschnitt, welcher den zweiten Abschnitt bildet, auf, wobei die Klingenabschnitte jeweils radial von der Drehachse längserstreckt abragen und entgegengesetzt orientierte Schneiden aufweisen. Bei dieser Ausgestaltung der Erfindung weist das Exzenterelement eine exzentrische, im weitesten Sinne längliche Kontur auf. Diese wird abschnittsweise durch den ersten Klingenabschnitt und den zweiten Klingenabschnitt gebildet. Beim Verspannen des Exzenterelements dringen die Klingenabschnitte, genauer: deren Schneiden, jeweils in die zugeordnete Knochenstruktur ein. Im Speziellen tritt die Schneide des ersten Klingenabschnitts in den Außenumfang des Knochendeckels ein. Die Schneide des zweiten Klingenabschnitts tritt in den Innenumfang des Schädelknochens ein. Hierdurch wird eine besonders zuverlässige Fixierung erreicht. Bei unterschiedlichen Ausgestaltungen sind unterschiedliche Anzahlen an Klingenabschnitten vorhanden. Beispielsweise sieht eine Ausgestaltung vor, dass mehrere Klingenabschnitte paarweise in - in Bezug auf die Längsachse übereinander angeordneten - Ebenen angeordnet sind.

In weiterer Ausgestaltung der Erfindung weist der Mechanismus ein Druckelement und ein Federelement auf, wobei das Druckelement zwischen einem ersten Stirnende, welches den ersten Abschnitt oder den zweiten Abschnitt bildet, und einem zweiten Stirnende entlang der Querachse längserstreckt und beweglich gelagert ist, wobei das Federelement wenigstens mittelbar an dem zweiten Stirnende abgestützt ist, und wobei das Druckelement mittels des Federelements entlang der Querachse federkraftbelastet vorgespannt ist. Je nach Orientierung des Druckelements und Anordnung des Federelements bildet das erste Stirnende des Druckelements entweder den ersten Abschnitt oder den zweiten Abschnitt. Folglich ist das Druckelement mittels des Federelements entweder an den Knochendeckel oder an den umrandenden Schädelknochen anpressbar. Die Federkraft des Federelements wirkt entlang der Querachse. Zur Fixierung kann das Druckelement, beispielsweise manuell, entgegen der Federkraft entlang der Querachse verlagert und hierdurch der projizierte Querabstand zwischen dem ersten Abschnitt und dem zweiten Abschnitt verringert werden. Nach erfolgter Vorpositionierung kann der manuelle Druck auf das Druckelement gelöst werden, wodurch die Feder das Druckelement gegen die entsprechende Knochenstruktur anpresst. Bei einer alternativen Ausgestaltung ist das Druckelement in einer in Bezug auf die Querachse hinteren Endlage lösbar entgegen der Federkraftbeaufschlagung arretiert. Nach einem Lösen der Arretierung drückt die Federkraft das Druckelement entlang der Querachse an die entsprechende Knochenstruktur.

In weiterer Ausgestaltung der Erfindung ist ein Arretierelement vorhanden, welches relativ zu dem Druckelement verlagerbar ist zwischen einer Arretierposition, in welcher das Druckelement mittels des Arretierelements entgegen der Federkraft des Federelements in Bezug auf die Querachse festgelegt ist, und einer Freigabeposition, in welcher die Beweglichkeit des Druckelements entlang der Querachse freigegeben ist. Die Verlagerung des Arretierelements zwischen der Arretier- und der Freigabeposition erfolgt vorzugsweise manuell. In der Arretierposition ist ein Abschnitt des Arretierelements lösbar form- und/oder kraftschlüssig mit einem Abschnitt des Druckelements verbunden. Durch eine Verlagerung in Richtung der Freigabeposition wird die besagte Verbindung zwischen dem Arretier- und dem Druckelement gelöst. Nach Lösen der Verbindung ist die Beweglichkeit des Druckelements entlang der Querachse freigegeben, so dass die Federkraft zu einem Anpressen des ersten Stirnendes an die je nach Ausgestaltung zugeordnete Knochenstruktur (Knochendeckel oder Schädelknochen) führt.

In weiterer Ausgestaltung der Erfindung weist der Mechanismus ein erstes Zylinderelement und ein zweites Zylinderelement auf, deren Längsachsen koaxial und jeweils parallel zu der Querachse ausgerichtet sind, wobei das erste Zylinderelement eine profilierte erste Zylindermantelfläche und eine den ersten Abschnitt bildende erste Fläche aufweist, wobei das zweite Zylinderelement eine komplementär profilierte zweite Zylindermantelfläche und eine den zweiten Abschnitt bildende zweite Fläche aufweist, wobei die erste Zylindermantelfläche und die zweite Zylindermantelfläche unter Ausbildung einer entlang der Querachse form- und/oder kraftschlüssigen Verbindung in unterschiedlichen axialen Relativpositionen der Zylinderelemente miteinander verbindbar sind. Unterschiedliche axiale Relativpositionen bewirken einen unterschiedlichen Querabstand zwischen der ersten Fläche und der zweiten Fläche. Mit anderen Worten ausgedrückt sind daserste Zylinderelement und zweite Zylinderelemententlang der Querachse miteinander zusammengefügt. Bei einer Ausgestaltung ist das erste Zylinderelement in das zweite Zylinderelement eingefügt, wobei in diesem Fall die profilierte erste Zylindermantelfläche eine Außenmantelfläche und die profilierte zweite Zylindermantelfläche eine Innenmantelfläche ist. Bei einer weiteren Ausgestaltung ist das zweite Zylinderelement in das erste Zylinderelement eingefügt, wobei die profilierte erste Zylindermantelfläche eine Innenmantelfläche und die profilierte zweite Zylindermantelfläche eine Außenmantelfläche ist. In beiden Fällen fungiert eines der beiden Zylinderelemente als eine Art Buchse und das verbleibende Zylinderelement als eine Art Bolzen, Stift oder dergleichen. Die an den Zylindermantelflächen vorhandenen Profilierungen können jeweils insbesondere als Rastgeometrie oder Gewindegeometrie ausgebildet sein. Dementsprechend ist die Verbindung bei einer Ausgestaltung eine Rastverbindung und bei einer weiteren Ausgestaltung eine Schraubverbindung. Bei letztgenannter Ausgestaltung werden die beiden Zylinderelemente zur Vergrößerung des Querabstands auseinandergeschraubt, aber selbstverständlich nicht vollständig voneinander gelöst. Bei der Ausgestaltung mit Rastverbindung wird letztere zur Vergrößerung des Querabstands einseitig überrastet.

In weiterer Ausgestaltung der Erfindung ist die zweite Fläche eine Stirnendfläche des zweiten Zylinderelements und zur radialen Anlage an dem Innenumfang des Schädelknochens eingerichtet, und die erste Fläche ist an der Außenmantelfläche des ersten Zylinderelements und zum Einfügen in eine radial in den Außenumfang des Knochendeckels erstreckte Bohrung eingerichtet. Bei dieser Ausgestaltung wird das Implantat zwecks Vorfixierung zunächst mit dem Knochendeckel zusammengefügt. Zu diesem Zweck wird die Außenmantelfläche des ersten Zylinderelements in die an dem Knochendeckel vorbereitete Radialbohrung eingesteckt. Hierbei kann das erste Zylinderelement entweder lose oder kraft-, form- und/oder stoffschlüssig in die Radialbohrung eingefügt werden. Nach erfolgter Vorfixierung kann der Knochendeckel gemeinsam mit dem an ihm angebrachten Implantat in der kranialen Öffnung positioniert werden. Zur eigentlichen Fixierung werden die beiden Zylinderelemente entlang der Querachse relativ zueinander verlagert. Dies beispielsweise über die zuvor beschriebene Rast- oder Schraubverbindung. Die Relativverlagerung bewirkt eine Vergrößerung des Querabstands, so dass der Ringspalt im Bereich des Implantats vergrößert, auf einer diametral gegenüberliegenden Seite verringert und schlussendlich unter Ausbildung eines Knochen-Knochen-Kontakts zwischen dem Knochendeckel und dem umrandenden Schädelknochen vollständig geschlossen wird.

In weiterer Ausgestaltung der Erfindung ist die erste Zylindermantelfläche eine Außenmantelfläche und die zweite Zylindermantelfläche ist eine Innenmantelfläche. Bei dieser Ausgestaltung ist das erste Zylinderelement folglich in das zweite Zylinderelement eingefügt. Zur Veränderung des Querabstands zwischen den gegenüberliegenden Stirnendflächen der Zylinderelemente, d.h. dem ersten und dem zweiten Abschnitt, kann das erste Zylinderelement axial weiter in das zweite Zylinderelement hineinverlagert oder axial aus diesem herausverlagert werden.

In weiterer Ausgestaltung der Erfindung weisen die erste Zylindermantelfläche und die zweite Zylindermantelfläche jeweils eine Profilierung in Form einer Rastgeometrie auf und sind entlang der Querachse einseitig überrastbar miteinander verrastet. Die Rastgeometrie der ersten Zylindermantelfläche ist komplementär zu der Rastgeometrie der zweiten Zylindermantelfläche und umgekehrt. Einseitig überrastbar meint, dass die zwischen den Rastgeometrien ausgebildete Rastverbindung entlang der Querachse in eine Richtung stets formschlüssig wirkt, so dass eine Querbewegung in diese Richtung vollständig gesperrt ist. In die entgegengesetzte Richtung ist die Rastverbindung überrastbar, so dass die beiden Zylinderelemente in unterschiedlichen axialen Relativpositionen miteinander verbindbar sind.

In weiterer Ausgestaltung der Erfindung weisen die erste Zylindermantelfläche und die zweite Zylindermantelfläche jeweils eine Profilierung in Form einer Gewindegeometrie auf und sind entlang der Querachse gewindebeweglich miteinander verschraubt.

Die Erfindung betrifft zudem ein Implantatsystem mit wenigstens zwei Implantaten gemäß der vorhergehenden Beschreibung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch stark vereinfachter Perspektivdarstellung den Kopf eines Patienten, wobei der knöcherne Schädel des Patienten eine mit einem Knochendeckel verschlossene kraniale Öffnung aufweist,
- Fig. 2: eine schematisch vereinfachte Querschnittsdarstellung des Schädels nach Fig. 1 im Bereich der kranialen Öffnung, wobei der Knochendeckel zeichnerisch ausgeblendet ist,
- Fig. 3: der anhand Fig. 2 dargestellte Bereich unter Einblendung des Knochendeckels,
- Fig. 4: der anhand der Fig. 2 und 3 gezeigte Bereich, mit einer Ausführungsform eines erfindungsgemäßen Implantats zur Fixierung des Knochendeckels in der kranialen Öffnung,
- Fig. 5: in schematischer Perspektivdarstellung eine weitere Ausführungsform eines erfindungsgemäßen Implantats,
- Fig. 6: eine schematische Längsschnittdarstellung des Implantats nach Fig. 5,
- Fig. 7: eine schematische Aufsicht des Implantats nach den Fig. 5 und 6,
- Fig. 8: eine schematische Seitenansicht des Implantats nach den Fig. 5 bis 7,
- Fig. 9: eine weitere, um 90° gedrehte Seitenansicht des Implantats nach den Fig. 5 bis 8,
- Fig. 10: in schematisch stark vereinfachter Darstellung eine erste intraoperative Situation unter Verwendung des Implantats nach den Fig. 5 bis 9,
- Fig. 11: eine zweite intraoperative Situation unter Verwendung des Implantats nach den Fig. 1 bis 9,
- Fig. 12: die zweite intraoperative Situation nach Fig. 11 in einer schematischen Schnittansicht entlang eines Schnitts XII-XII,
- Fig. 13: in schematischer Perspektivdarstellung eine erste Variante des Implantats nach den Fig. 5 bis 9,
- Fig. 14: in schematischer Perspektivdarstellung eine zweite Variante des Implantats nach den Fig. 5 bis 9,
- Fig. 15: in schematischer Schnittansicht eine dritte Variante des Implantats nach den Fig. 5 bis 9,
- Fig. 16: in schematischer Seitenansicht eine vierte Variante des Implantats nach den Fig. 5 bis 9,
- Fig. 17: in schematischer Perspektivdarstellung eine weitere Ausführungsform eines erfindungsgemäßen Implantats,
- Fig. 18: das Implantat nach Fig. 17 in einer schematischen Seitenansicht,
- Fig. 19: eine um 90° gedrehte weitere Seitenansicht des Implantats nach den Fig. 17 und 18,
- Fig. 20: eine schematische Längsschnittdarstellung des Implantats nach den Fig. 17 bis 19,
- Fig. 21: in vergrößerter Detaildarstellung einen Bereich XXI des Implantats nach den Fig. 17 bis 20,
- Fig. 22: eine intraoperative Situation unter Verwendung des Implantats nach den Fig. 17 bis 21,
- Fig. 23: in schematischer Perspektivdarstellung eine erste Variante des Implantats nach den Fig. 17 bis 21,
- Fig. 24: in schematischer Perspektivdarstellung eine zweite Variante des Implantats nach den Fig. 17 bis 21,
- Fig. 25: in schematischer Perspektivdarstellung eine dritte Variante des Implantats nach den Fig. 17 bis 21 unter zeichnerischer Ausblendung einzelner Bauteile und/oder Abschnitte des Implantats,
- Fig. 26: in schematischer Perspektivdarstellung eine vierte Variante des Implantats nach den Fig. 17 bis 21,
- Fig. 27: in schematischer Perspektivdarstellung eine fünfte Variante des Implantats nach den Fig. 17 bis 21,
- Fig. 28, 29: eine sechste Variante des Implantats nach den Fig. 17 bis 21 in einer schematischen Perspektivdarstellung (Fig. 28) und einer schematischen Aufsicht (Fig. 29),
- Fig. 30: in schematischer Perspektivdarstellung eine weitere Ausführungsform eines erfindungsgemäßen Implantats,
- Fig. 31: das Implantat nach Fig. 30 in einer schematischen Aufsicht,
- Fig. 32: eine schematische Längsschnittdarstellung des Implantats nach den Fig. 30 und 31,
- Fig. 33: in einer Schnittdarstellung entsprechend Fig. 12 eine intraoperative Situation unter Verwendung des Implantats nach den Fig. 30 bis 32,
- Fig. 34: in schematischer sowie teiltransparenter Perspektivdarstellung eine erste Variante des Implantats nach den Fig. 30 bis 32,
- Fig. 35: eine zweite Variante des Implantats nach den Fig. 30 bis 32 in schematischer sowie teiltransparenter Perspektivdarstellung,
- Fig. 36: in schematischer Perspektivdarstellung eine weitere Ausführungsform eines erfindungsgemäßen Implantats mit einem ersten Zylinderelement und einem zweiten Zylinderelement,
- Fig. 37: in schematischer Perspektivdarstellung das erste Zylinderelement des Implantats nach Fig. 36,
- Fig. 38: in schematischer Perspektivdarstellung das zweite Zylinderelement des Implantats nach Fig. 36,
- Fig. 39: das Implantat nach Fig. 36 in einer schematischen Längsschnittdarstellung,
- Fig. 40: in schematischer Perspektivdarstellung eine Variante des zweiten Zylinderelements nach Fig. 38,
- Fig. 41: in schematisch stark vereinfachter Darstellung eine intraoperative Situation unter Verwendung des Implantats nach den Fig. 36 bis 39,
- Fig. 42: in schematisch stark vereinfachter Seitenansicht eine erste Variante des Implantats nach den Fig. 36 bis 39,
- Fig. 43: eine zweite Variante des Implantats nach den Fig. 36 bis 39 in schematisch stark vereinfachter Seitenansicht,
- Fig. 44: eine schematische Seitenansicht einer dritten Variante des Implantats nach den Fig. 36 bis 39,
- Fig. 45: in schematischer Seitenansicht eine vierte Variante des Implantats nach den Fig. 36 bis 39,
- Fig. 46: in schematischer Darstellung eine weitere intraoperative Situation unter Verwendung des Implantats nach den Fig. 36 bis 39 und eines Pins,
- Fig. 47: in schematischer Perspektivdarstellung der Pin nach Fig. 46 und
- Fig. 48: in schematischer Perspektivdarstellung eine Variante des Pins nach Fig. 47.

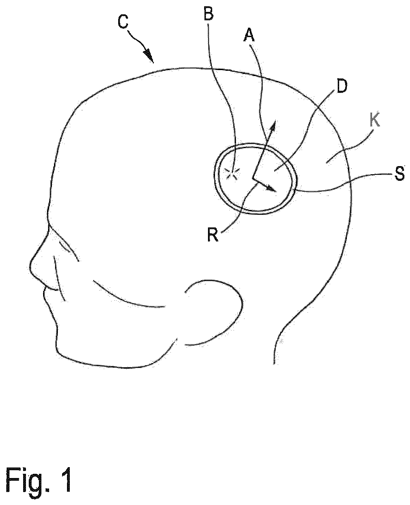

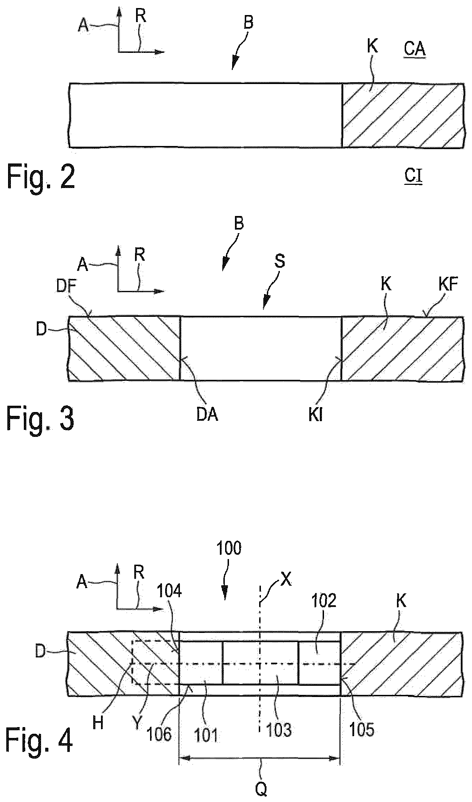

Gemäß Fig. 1 weist der Kopf eines Patienten eine kraniale Öffnung B auf, welche ausgehend von einer Schädelaußenseite CA durch den knöchernen Schädel C des Patienten bis auf eine Schädelinnenseite CI erstreckt ist (Fig. 2). Die kraniale Öffnung B ist entlang ihrer Axialrichtung A durch den knöchernen Schädel C erstreckt. Entlang ihrer Radialrichtung R ist die kraniale Öffnung B von Schädelknochen K berandet.

Die kraniale Öffnung kann grundsätzlich durch einen Unfall oder eine sonstige unbeabsichtigte Einwirkung bedingt sein. Im vorliegenden Fall ist die kraniale Öffnung B das Ergebnis einer Kraniotomie und dient als chirurgisch hergestellter Zugang für einen neurochirurgischen Eingriff am Gehirn des Patienten.

Bei einer solchen Kraniotomie wird der knöcherne Schädel C durch ein mechanisches Heraustrennen eines Knochendeckels D geöffnet. Dies kann beispielsweise mittels einer Trepanation erfolgen. Hierbei wird der Knochendeckel D unter Ausbildung eines Ringspalts S aus dem knöchernen Schädel C herausgetrennt. Der Ringspalt S wird auch als Kraniotomiespalt bezeichnet. Der Knochendeckel D kann auch als Knochenteil, Knochenfragment oder Knochensegment bezeichnet werden.

Nach dem Heraustrennen des Knochendeckels D kann dieser unter Ausbildung der eigentlichen kranialen Öffnung B vorläufig von dem übrigen knöchernen Schädel entfernt werden. Nach erfolgtem Eingriff am Gehirn wird der Knochendeckel D in der Öffnung B repositioniert und fixiert.

Gemäß Fig. 4 ist ein Implantat 100 zur Fixierung des kranialen Knochendeckels D in der kranialen Öffnung B vorgesehen. Das Implantat 100 weist eine Längsachse X, eine Querachse Y, einen ersten Abschnitt 101, einen zweiten Abschnitt 102 und einen Mechanismus 103 auf.

Die Längsachse X ist entlang der Axialrichtung A der kranialen Öffnung B ausgerichtet. Die orthogonal zu der Längsachse X orientierte Querachse Y ist entlang der Radialrichtung R der kranialen Öffnung B ausgerichtet. Die besagten Ausrichtungen der Längsachse X und der Querachse Y beziehen sich auf die anhand Fig. 4 gezeigte Konfiguration, in welcher der Knochendeckel D mittels des Implantats 100 fixiert ist.

Der erste Abschnitt 101 ist zur Kraftübertragung auf den Knochendeckel D eingerichtet. Dementsprechend ist der erste Abschnitt 101 dem Knochendeckel D zugewandt.

Der zweite Abschnitt 102 ist zur Kraftübertragung auf den die kraniale Öffnung B umrandenden Schädelknochen K eingerichtet. Dementsprechend ist der zweite Abschnitt dem Schädelknochen K zugewandt.

Der Mechanismus 103 ist mit dem ersten Abschnitt 101 und mit dem zweiten Abschnitt 102 wirkverbunden und zur Veränderung eines auf die Querachse Y projizierten Querabstands Q zwischen dem ersten Abschnitt 101 und dem zweiten Abschnitt 102 eingerichtet. Durch die besagte Vergrößerung des Querabstands Q kann der erste Abschnitt 101 in Radialrichtung R nach innen an den Knochendeckel D angepresst werden und der zweite Abschnitt 102 kann in Radialrichtung R nach außen an den Schädelknochen K angepresst werden. Die Kraftübertragung zwischen dem ersten Abschnitt 101 und dem Knochendeckel D einerseits und dem zweiten Abschnitt 102 und dem Schädelknochen K andererseits erfolgt entlang der Radialrichtung R, so dass auch von einer radialen Fixierung gesprochen werden kann.

Zur Fixierung des Knochendeckels D wird das Implantat 100 entlang der Axialrichtung A in den Ringspalt S eingebracht. Der erste Abschnitt 101 wird in Radialrichtung R nach innen orientiert, d.h. in Richtung des Knochendeckels D. Der zweite Abschnitt 102 wird in Radialrichtung R nach außen orientiert, d.h. in Richtung des Schädelknochens K. Hiernach wird der Mechanismus 103 zur Vergrößerung des Querabstands Q betätigt. Diese Betätigung kann direkt oder indirekt unter Zuhilfenahme eines Werkzeugs oder rein manuell erfolgen. Die Betätigung des Mechanismus bewirkt die besagte Vergrößerung des Querabstands Q. Infolge der Vergrößerung des Querabstands Q wirkt der erste Abschnitt 101 in Radialrichtung R nach innen auf den Knochendeckel D und der zweite Abschnitt 102 wirkt in Radialrichtung R entgegengesetzt nach außen auf den Schädelknochen K. Hierdurch wird der Ringspalt S lokal, nämlich im Bereich des Implantats 100, vergrößert. Die lokale Vergrößerung des Ringspalts S geht zwangsläufig mit einer lokalen Verringerung des Ringspalts S auf einer dem Implantat in Radialrichtung R 100 diametral gegenüberliegenden Seite des Knochendeckels D einher. In diesem Bereich wird der Knochendeckel D unter Einwirkung des Implantats 100 in Radialrichtung R gegen den Schädelknochen K gedrückt. Hierdurch wird ein Knochen-Knochen-Kontakt etabliert und der Knochendeckel D wird über eine Verspannung in Radialrichtung R in der Öffnung B fixiert.

Prinzipiell genügt es, wenn ein einziges Implantat zur Fixierung verwendet wird. Selbstverständlich kann jedoch auch mehr als ein Implantat verwendet werden. Sofern mehrere Implantate verwendet werden, werden diese in Umfangsrichtung des Ringspalts S voneinander beabstandet in demselben angeordnet. Die mehreren Implantate werden dann jeweils über eine Vergrößerung ihres projizierten Querabstands Q zwischen dem Knochendeckel D und dem umrandenden Schädelknochen K radial verspannt. Eine Fixierung mittels mehrerer Implantate wird nachfolgend noch näher beschrieben werden.

Der Mechanismus ist bei unterschiedlichen Ausführungsformen unterschiedlich ausgestaltet. Dabei sind zum Vergrößern des Querabstands Q, d.h. zum Generieren der Spreiz- oder auch Aufspreizbewegung zwischen den beiden Abschnitten unterschiedlichste Konstruktionen denkbar. Beispielsweise kann der Mechanismus auf einem Schraub- oder Gewindeprinzip, einem Scherengelenkmechanismus, einem Keil-, Kegel- und/oder Klemmmechanismus, einem Rastmechanismus, einem Exzentermechanismus oder dergleichen beruhen. Dabei kann die mittels des Mechanismus bewirkbare Vergrößerung des Querabstands Q auf einer translatorischen und/oder rotatorischen Verlagerung des ersten Abschnitts und/oder des zweiten Abschnitts basieren. Beispielsweise kann der erste Abschnitt entlang der Querachse Y radial nach innen verlagerbar sein, wobei der zweite Abschnitt in Bezug auf die Querachse Y feststeht oder umgekehrt. Entsprechendes gilt mutatis mutandis hinsichtlich einer rotatorischen Verlagerung um die Längsachse X.

Die Kraftübertragung zwischen den beiden Abschnitten und der jeweils zugeordneten Knochenstruktur, d.h. dem Knochendeckel D einerseits und dem Schädelknochen K andererseits, kann direkt oder indirekt erfolgen. Direkt meint, dass ein unmittelbarer Kontakt zwischen dem jeweiligen Abschnitt und der entsprechenden Knochenstruktur etabliert wird. Indirekt meint, dass weitere Bauteile oder Komponenten des Implantats zwischen dem jeweiligen Abschnitt und der jeweils zugeordneten Knochenstruktur D, K angeordnet sein können.

Dabei kann die Kraftübertragung auf den Knochendeckel D zum einen auf dessen Außenumfang DA erfolgen. Alternativ oder zusätzlich kann die Kraft über eine in den Knochendeckel D eingebrachte Bohrung H erfolgen, wie sie strichliert in Fig. 4 angedeutet ist. Für die Kraftübertragung auf den Schädelknochen K gilt Entsprechendes sinngemäß. Das heißt die Kraftübertragung kann zum einen auf den Innenumfang KI des Schädelknochens K erfolgen. Prinzipiell denkbar ist zudem, dass eine Bohrung oder dergleichen in radialer Richtung R in den Schädelknochen K zwecks Kraftübertragung eingebracht wird.

Bei dem Implantat 100 nach Fig. 4 erfolgt die Kraftübertragung direkt auf den jeweiligen Umfang der Knochenstruktur. Folglich weist der erste Abschnitt 101 eine erste Kontaktfläche 104 auf. Diese ist zur (unmittelbaren) radialen Anlage an dem Außenumfang DA des Knochendeckels D eingerichtet. Der zweite Abschnitt 102 weist eine zweite Kontaktfläche 105 auf. Diese ist zur (unmittelbaren) radialen Anlage an dem Innenumfang KI des Schädelknochens K eingerichtet. Eine nicht näher bezeichnete Normalenrichtung der ersten Kontaktfläche 104 weist vorliegend parallel zur Querachse Y sowie radial nach innen. Eine nicht näher bezeichnete Normalenrichtung der zweiten Kontaktfläche 105 weist vorliegend parallel zu der Querachse Y sowie radial nach außen. Folglich sind die Kontaktflächen 104, 105 zueinander entgegengesetzt orientiert.

Weiter in Bezug auf Fig. 4 weist der erste Abschnitt 101 eine Fügefläche 106 auf. Die Fügefläche 106 kommt insbesondere dann zum Tragen, wenn der Knochendeckel D mit einer Bohrung H versehen ist. In diesem Fall kann der Abschnitt 101, genauer: dessen Fügefläche 106, in die Bohrung H eingefügt werden. Die Kraftübertragung erfolgt in diesem Fall nicht auf den Außenumfang DA, sondern stattdessen über die nicht näher bezeichneten Wandungen der Bohrung H.

Bei dem Implantat 100 nach Fig. 4 sind der erste Abschnitt 101, der zweite Abschnitt 102 und der Mechanismus 103 vollständig innerhalb des Ringspalts S angeordnet. In Bezug auf die Radialrichtung R und damit auch die Querrichtung Y ist das gesamte Implantat 100 zwischen dem Außenumfang DA des Knochendeckels D und dem Innenumfang KI des Schädelknochens K angeordnet. In Bezug auf die Axialrichtung A und damit auch auf die Längsachse X ist das gesamte Implantat 100 zwischen der Schädelaußenseite CA und der Schädelinnenseite CI angeordnet. Folglich ragt das Implantat 100 entlang der Längsachse X weder nach oben noch nach unten aus dem Ringspalt S heraus.

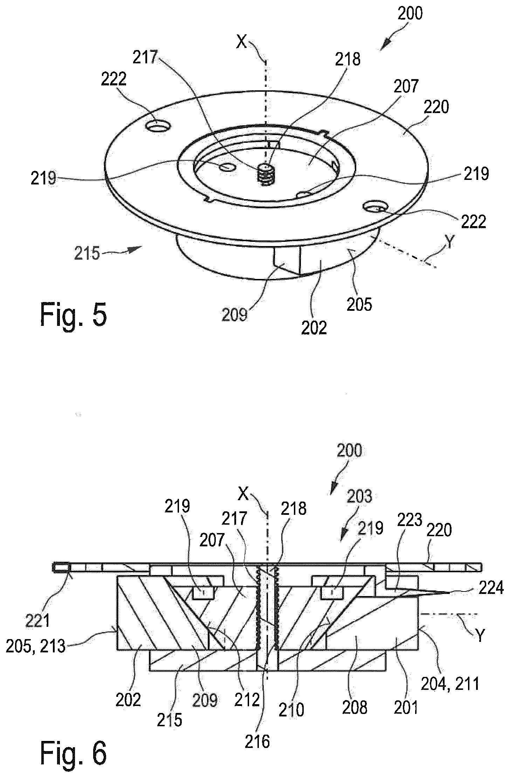

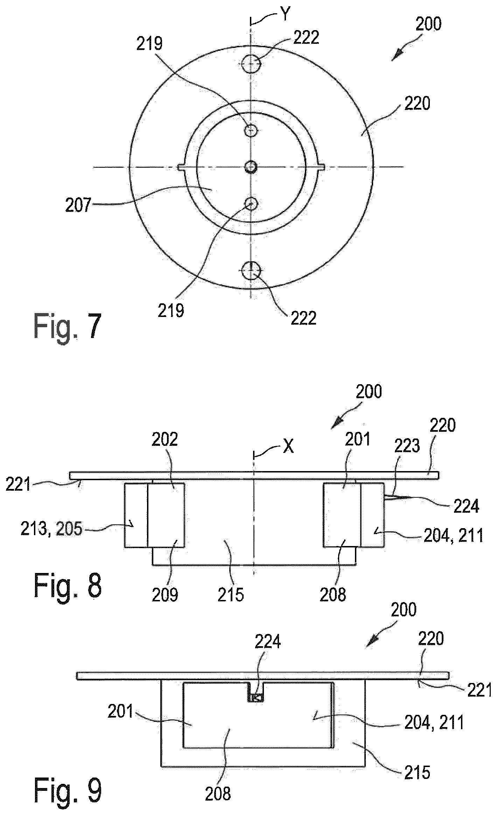

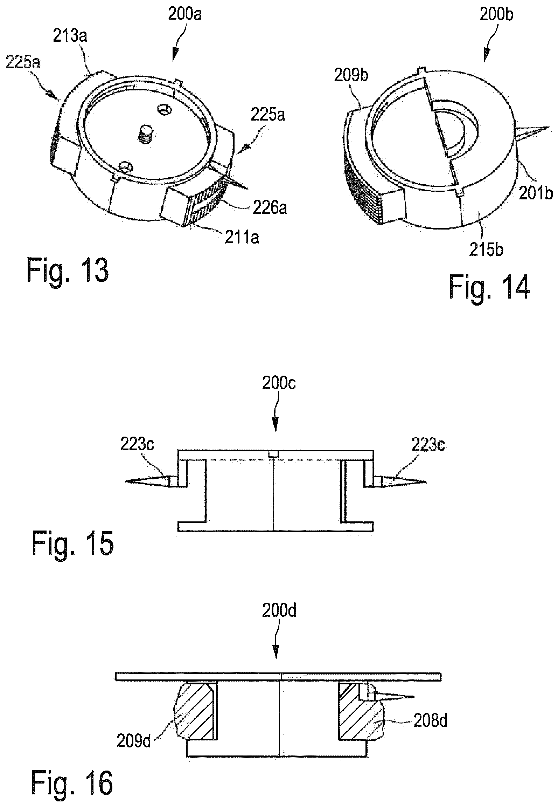

Die Fig. 5 bis 16 zeigen eine weitere Ausführungsform eines erfindungsgemäßen Implantats 200, wobei die Fig. 13 bis 16 Varianten dieser Ausführungsform betreffen.

Mit Bezug auf die Fig. 5 bis 9 weist das Implantat 200 eine Längsachse X, eine Querachse Y, einen ersten Abschnitt 201, einen zweiten Abschnitt 202 und einen Mechanismus 203 auf. Der Mechanismus 203 weist ein Kegelelement 207, ein erstes Spannelement 208 und ein zweites Spannelement 209 auf.

Die beiden Spannelemente 208, 209 sind jeweils entlang der Querachse Y beweglich gelagert.

Das erste Spannelement 208 weist eine erste Innenkonusfläche 210 und eine erste Stirnfläche 211 auf. Die erste Innenkonusfläche 210 liegt in Bezug auf die Längsachse X innen, die erste Stirnfläche 211 dementsprechend außen. Die erste Stirnfläche 211 ist zur radialen Anlage an dem Außenumfang DA des Knochendeckels D eingerichtet und fungiert insoweit als erste Kontaktfläche 204. Das erste Spannelement 208 und/oder dessen erste Stirnfläche 211 bildet mithin den ersten Abschnitt 201 des Implantats 200.

Das zweite Spannelement 209 weist eine zweite Innenkonusfläche 212 und eine zweite Stirnfläche 213 auf. Die zweite Stirnfläche 213 liegt in Bezug auf die Längsachse X außen, die zweite Innenkonusfläche 212 liegt innen. Die zweite Stirnfläche 213 ist zur radialen Anlage an dem Innenumfang KI des Schädelknochens K eingerichtet und dient mithin als zweite Kontaktfläche 205. Das zweite Spannelement 209 und/oder dessen zweite Stirnfläche 213 bildet folglich den zweiten Abschnitt 202 des Implantats 200.

Das Kegelelement 207 ist entlang der Längsachse X beweglich gelagert und weist eine mit den beiden Innenkonusflächen 210, 212 gleitbeweglich zusammenwirkende Außenkonusfläche 214 auf. Das Kegelelement 207 ist in Bezug auf die Längsachse X zwischen einer in den Figuren nicht gezeigten oberen Endlage und einer unteren Endlage (Fig. 6) verlagerbar. In der oberen Endlage sind die beiden Spannelemente 208, 209 jeweils entlang der Querachse Y und in Bezug auf die Längsachse X nach innen aufeinander zubewegt. In der oberen Endlage liegt folglich ein nicht näher dargestellter erster Querabstand zwischen der ersten Kontaktfläche 204 und der zweiten Kontaktfläche 205 vor. Eine Längsverlagerung des Spannelements 207 ausgehend von der oberen Endlage in Richtung der unteren Endlage (Fig. 6) bewirkt, dass die Spannelemente 208, 209 jeweils unter Vergrößerung des Querabstands Q entlang der Querachse Y nach außen verlagert werden. Dabei wird die Längsbewegung des Kegelelements 207 über das Zusammenwirken der Außenkonusfläche 214 mit den beiden Innenkonusflächen 210, 212 in die jeweilige Querbewegung der Spannelemente 208, 209 übersetzt. Das Übersetzungsverhältnis kann konstruktiv über eine Anpassung der jeweiligen Neigung bzw. des Konuswinkels der Konusflächen 210, 212, 214 verändert werden.

Zur Aufnahme und/oder beweglichen Lagerung der beiden Spannelemente 208, 209 weist das Implantat 200 vorliegend ein Gehäuse 215 auf. Das Gehäuse 215 weist eine hohlzylindrische Gestalt mit einem kreisringförmigen Querschnitt auf und ist an einander in Querrichtung Y gegenüberliegenden Seiten mit nicht näher bezeichneten Aufnahmeöffnungen versehen, in welche die Spannelemente 208, 209 eingebracht sind. Die Aufnahmeöffnungen können jeweils auch als Tasche bezeichnet werden.

Das Kegelelement 207 ist bei der gezeigten Ausführungsform gewindebeweglich entlang der Längsachse X gelagert. Zu diesem Zweck weist das Kegelelement 207 ein Gewinde 216 auf, welches mit einem komplementären und feststehenden Gegengewinde 217 verschraubt ist. Das Gewinde 216 ist vorliegend ein Innengewinde. Dementsprechend ist das Gegengewinde 217 ein Außengewinde. Das Gegengewinde 217 ist bei der gezeigten Ausführungsform an einem parallel, genauer: koaxial, zu der Längsachse X längserstreckten Gewindestab 218 ausgebildet. Der Gewindestab 218 ist an seinem in Bezug auf die Längsachse X unteren Stirnende an dem Gehäuse 215 festgelegt.

Bei der gezeigten Ausführungsform ist das Kegelelement 207 zur Kraft- und/oder Drehmomentbeaufschlagung mittels eines hierfür geeigneten Werkzeugs eingerichtet und weist dementsprechende Werkzeugaufnahmen 219 auf. Die Werkzeugaufnahmen 219 sind ausgehend von einer nicht näher bezeichneten Oberseite des Kegelelements 207 entlang der Längsachse X eingesenkt und weisen vorliegend eine kreiszylindrische Querschnittsform auf. Die Werkzeugaufnahmen 219 sind in Umfangsrichtung um 180° zueinander versetzt angeordnet.

Zum Aufspreizen der beiden Spannelemente 208, 209 - d.h. zur Vergrößerung des (projizierten) Querabstands - wird das besagte Werkzeug lösbar mit den Werkzeugaufnahmen 219 verbunden und ein um die Längsachse X gerichtetes Drehmoment aufgebracht. Unter Einwirkung des Drehmoments kann das Kegelelement 207 entlang des Gewindestabs 218 nach unten geschraubt werden, wodurch die Spannelemente 208, 209 jeweils aus dem Gehäuse 215 herausbewegt und insoweit gespreizt werden.

Bei der gezeigten Ausführungsform weist das Implantat 200 zudem einen Stützabschnitt 220 auf, welcher entlang der Längsachse X oberhalb der beiden Spannelemente 208, 209 und des Kegelelements 207 angeordnet ist. Insoweit ist der Stützabschnitt 220 oberhalb des ersten Abschnitts 201 und des zweiten Abschnitts 202 angeordnet. Der Stützabschnitt 220 weist eine Unterseite 221 auf, welche zum Abstützen des Implantats 200 auf einer Außenseite KF des Schädelknochens K einerseits und einer Außenseite DF des Knochendeckels D andererseits eingerichtet ist (siehe Fig. 2). Die Unterseite 221 ist orthogonal zu den Stirnseiten 211, 213 orientiert. Der Stützabschnitt 220 erlaubt eine vereinfachte Positionierung des Implantats 200 innerhalb des Ringspalts S. Durch die besagte Abstützung werden ein zu tiefes Eindringen in den Ringspalt S und damit einhergehende Risiken vermieden, beispielsweise eine druckbedingte Reizung der Dura mater, die schlechtestenfalls mit einem Epiduralhämatom einhergehen kann.

Der Stützabschnitt 220 ist bei der gezeigten Ausführungsform mittels einer lösbaren Fügeverbindung, genauer: einem Bajonettverschluss, mit dem Implantat 200 im Übrigen verbunden. Vorliegend ist der Bajonettverschluss zwischen einem nicht näher bezeichneten Innenumfang des Stützabschnitts 220 und einer oberseitig am dem Gehäuse 215 angeordneten Geometrie ausgebildet. Anstelle des Bajonettverschlusses kann beispielsweise auch eine übliche Schraubverbindung oder dergleichen vorgesehen sein. Alternativ denkbar ist eine Steck- oder Rastverbindung. Die lösbare Fügeverbindung ermöglicht, dass der Stützabschnitt 220 nach erfolgter Fixierung von dem Gehäuse 215 gelöst und entfernt werden kann. Nach dem Entfernen des Stützabschnitts 220 ist das (übrige) Implantat 200 in Bezug auf die Längsrichtung X vollständig innerhalb des Ringspalts S angeordnet. Dies hat kosmetische und medizinische Vorteile.

Bei der gezeigten Ausführungsform ist der Stützabschnitt 220 kreisscheibenförmig gestaltet und weist zwei um 180° in Umfangsrichtung zueinander versetzt angeordnete Werkzeugaufnahmen 222 auf. Die Werkzeugaufnahmen 222 dienen einer Aufnahme eines Werkzeugs zum Lösen und/oder Verbinden des besagten Bajonettverschlusses. Die Werkzeugaufnahmen 222 können alternativ oder zusätzlich als Bohrlöcher zur Vorfixierung des Implantats 200 an dem Knochendeckel D verwendet werden.

Vorliegend weist das Implantat 200 zudem einen Dornabschnitt 223 auf. Der Dornabschnitt 223 ist entlang der Querachse Y längserstreckt und weist eine einends über den ersten Abschnitt 201, d.h. vorliegend das erste Spannelement 208, hinausragende Dornspitze 224 auf. Diese ist zum radialen Einstechen in den Außenumfang DA des Knochendeckels D eingerichtet. Der Dornabschnitt 223 ist an seinem der Dornspitze 224 abgewandten Ende auf nicht näher gezeigte Weise an dem Gehäuse 215 festgelegt. Der Dornabschnitt 223 dient einer Vorfixierung zwischen Implantat 200 und Knochendeckel D. Zu diesem Zweck wird die Dornspitze 224 radial in den Knochendeckel D eingestochen. Dies geschieht vorzugsweise noch vor der Repositionierung des Knochendeckels D innerhalb der Öffnung B.

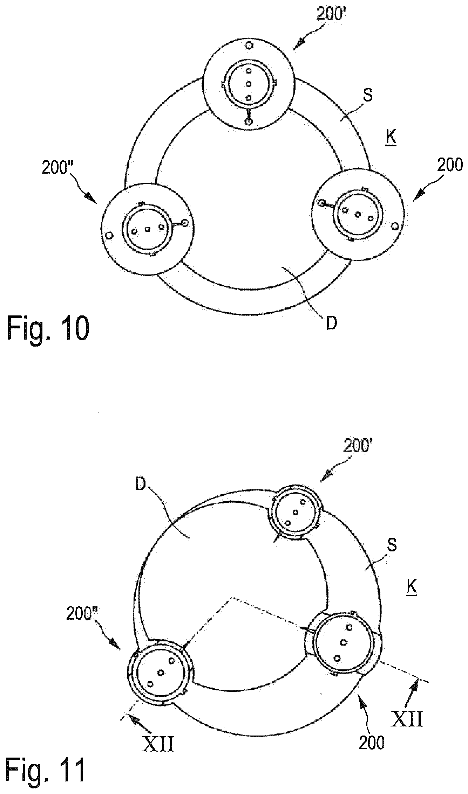

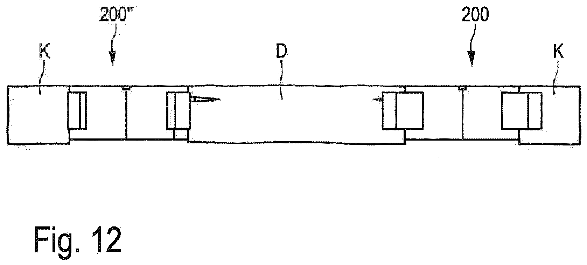

Die Fig. 10 bis 12 zeigen eine erste intraoperative Situation (Fig. 10) und eine zweite intraoperative Situation (Fig. 11, 12) unter Verwendung des Implantats 200. Die erste intraoperative Situation zeigt einen vorfixierten Zustand des Knochendeckels D, die zweite intraoperative Situation zeigt einen endgültig fixierten Zustand. Das gezeigte situative Beispiel sieht die Verwendung mehrerer baugleicher Implantate vor, nämlich des Implantats 200, eines zweiten Implantats 200' und eines dritten Implantats 200". Die Implantate 200, 200', 200" bilden ein Implantatsystem. Die Implantate 200, 200', 200" sind vorliegend in etwa um 120° in Umfangsrichtung des Knochendeckels D versetzt innerhalb des Ringspalts S angeordnet. Zwecks Vorfixierung ist der jeweilige Dornabschnitt der Implantate 200, 200', 200" auf die bereits beschriebene Weise in den Außenumfang DA des Knochendeckels D eingesteckt. Nach dem Ein- bzw. Zusammenstecken der Implantate 200, 200', 200" mit dem Knochendeckel D wird die insoweit vorfixierte Anordnung gemeinsam in der kranialen Öffnung B (re)positioniert. Hiernach ergibt sich die in Fig. 10 gezeigte Situation.

Zur eigentlichen Fixierung wird in dem gezeigten Beispiel das erste Implantat 200 expandiert. D.h. dessen Spannelemente 208, 209 werden über die bereits vorbeschriebene Betätigung des Kegelelements 207 in Radialrichtung R der Öffnung B aufgespreizt. Der Knochendeckel D wird hierdurch auf der dem ersten Implantat 200 diametral gegenüberliegenden Seite der Öffnung B an den umrandenden Schädelknochen K gepresst. Die weiteren Implantate 200', 200" dienen vorliegend im weitesten Sinne einer zusätzlichen Fixierung.

Im Übrigen ist anhand der Fig. 11 und 12 gezeigt, dass die jeweiligen Stützabschnitte der Implantate 200, 200', 200" nach erfolgter Fixierung entfernt sind. Hierdurch ragen die Implantate 200, 200', 200" nicht nach oben über die Schädelaußenseite CA hinweg.

Weiter in Bezug auf Fig. 11 ist erkennbar, dass die erste Kontaktfläche 204 des ersten Spannelements 208 sowie die zweite Kontaktfläche 205 des zweiten Spannelements 209 jeweils gekrümmt sind. Entsprechendes gilt hinsichtlich der weiteren Implantate 200', 200". Die besagte Krümmung ermöglicht eine möglichst vollflächige Kontaktierung und somit eine Übertragung möglichst großer Kontaktkräfte.

In der anhand Fig. 11 gezeigten Situation sind die Implantate 200, 200', 200" jeweils in einem sogenannten Bohrloch des Ringspalts S angeordnet. Die Bohrlöcher werden üblicherweise im Rahmen der Trepanation eingebracht und unter Zuhilfenahme eines hierfür geeigneten Werkzeugs miteinander verbunden. Durch die Verbindung der Bohrlöcher wird der eigentliche Ring- bzw. Kraniotomiespalt S ausgebildet. Die Krümmungsradien der Kontaktflächen 204, 205 sind vorliegend jeweils auf den Radius des Bohrlochs abgestimmt. Die Krümmungsradien der Kontaktflächen 204, 205 sind bei unterschiedlichen Ausführungsformen unterschiedlich und bewegen sich in einem Bereich zwischen 6 und 20 mm.

Die Fig. 13 bis 16 zeigen Varianten des Implantats 200 nach den Fig. 5 bis 9. Deren Funktion und/oder Aufbau ist weitestgehend identisch mit der Funktion und dem Aufbau des Implantats 200. Zur Vermeidung von Wiederholungen wird daher nachfolgend lediglich auf wesentliche Unterschiede der Varianten eingegangen. Funktionsgleiche Bauteile und/oder Abschnitte werden nicht gesondert erläutert. Stattdessen wird ausdrücklich auf das zu dem Implantat 200 Offenbarte verwiesen und Bezug genommen.

Das Implantat 200a zeigt eine unterschiedliche Gestaltung der Spannelemente. Deren Stirnseiten 211a, 213a sind jeweils mit einer Profilierung 225a versehen. Die Profilierung 225a dient einer Vergrößerung der jeweiligen Kontaktoberfläche. Die Profilierung 225a weist eine Vielzahl von Rillen auf, die parallel zu der Längsachse X längserstreckt und radial nach innen in die jeweilige Stirnfläche 211a, 213a eingesenkt sind. Selbstverständlich ist auch eine hiervon abweichende Gestaltung der Profilierung 225a denkbar. Bei einer nicht in den Figuren gezeigten Variante ist die Profilierung durch Waben, Zacken oder eine im weitesten Sinne aufgeraute Oberfläche gebildet.

Zur Gewichtsreduzierung weisen die Spannelemente des Implantats 200a zudem jeweils eine Aussparung 226a auf, die auch als Tasche bezeichnet werden kann. Die Aussparungen 226a sind jeweils ausgehend von der Stirnseite 211a, 213a entlang der Querachse Y nach innen in das jeweilige Spannelement eingesenkt.

Das Implantat 200b nach Fig. 14 weist im Unterschied lediglich ein Spannelement 209b auf. Bei dieser Variante fungiert der dem zweiten Spannelement 209b entlang der Querachse Y gegenüberliegende Randbereich des Gehäuses 215b als erster Abschnitt 201b.

Das Implantat 200c nach Fig. 15 sieht zwei Dornabschnitte 223c vor. Die Dornabschnitte 223c ragen zueinander entgegengesetzt entlang der Querachse Y nach außen ab. Die Variante mit zwei Dornabschnitten 223c ist insbesondere dann vorteilhaft, wenn der Knochendeckel aus mehreren, insbesondere fragmentierten, Segmenten besteht. Die den Knochendeckel bildenden Segmente oder Fragmente können unter Zuhilfenahme der Dornabschnitte 223c gleichsam "aneinandergepuzzelt" werden.

Bei der Variante nach Fig. 16 weist das Implantat 200d wiederum ein erstes Spannelement 208d und ein zweites Spannelement 209d auf. Die Spannelemente 208d, 209d sind bei dieser Variante aus einer plastisch deformierbaren Masse gefertigt, die unter Einwirkung des Kegelelements seitlich aus dem Gehäuse des Implantats 200d herausgepresst wird. Bei der plastisch deformierbaren Masse kann es sich um eine Art Knete oder dergleichen handeln.

Es versteht sich, dass die Merkmale des Implantats 200 sowie die anhand der Implantate 200a, 200b, 200c und 200d erläuterten Merkmale zu weiteren nicht in den Figuren gezeigten Merkmalskombinationen zusammenfassbar sind.

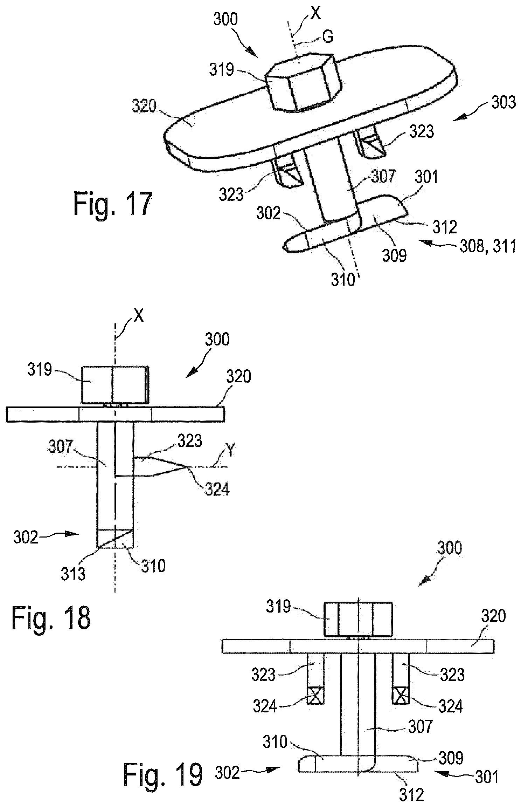

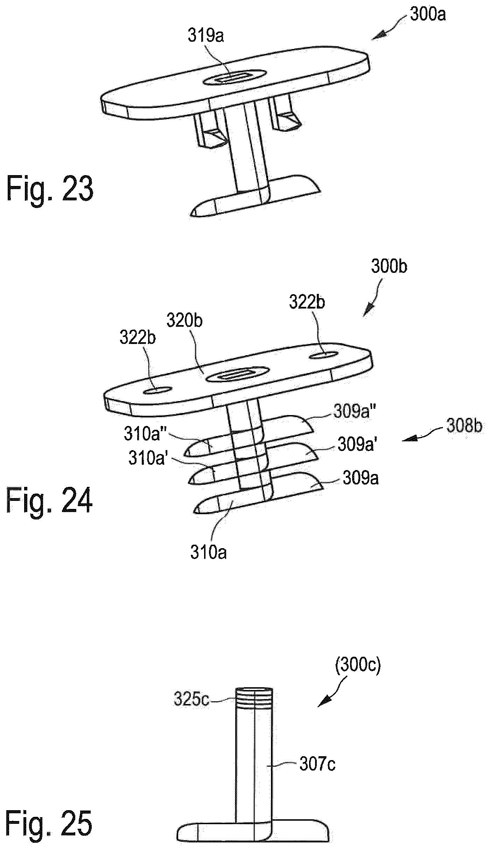

Die Fig. 17 bis 29 zeigen eine weitere Ausführungsform eines erfindungsgemäßen Implantats 300, wobei die Fig. 23 bis 29 Varianten dieser Ausführungsform betreffen.

Das Implantat 300 weist eine Längsachse X, eine Querachse Y, einen ersten Abschnitt 301, einen zweiten Abschnitt 302 und einen Mechanismus 303 auf. Der erste Abschnitt 301 ist wiederum zur Kraftübertragung auf den Knochendeckel D eingerichtet. Dementsprechend ist der zweite Abschnitt 302 zur Kraftübertragung auf den Schädelknochen K eingerichtet.

Der Mechanismus 303 ist - im Unterschied zu den zuvor erläuterten Ausführungsformen und/oder Varianten - zur rotatorischen Verlagerung des ersten Abschnitts 301 und des zweiten Abschnitts 302 eingerichtet.

Der Mechanismus 303 weist eine Drehachse G und ein Exzenterelement 308 auf.

Die Drehachse G ist bei der gezeigten Ausführungsform eine Achse im geometrischen Sinn und parallel, genauer: koaxial, zu der Längsachse X orientiert.

Das Exzenterelement 308 ist um die Drehachse G beweglich gelagert und weist eine in Bezug auf die Drehachse G exzentrische Kontur 311 auf. Unterschiedliche Abschnitte der exzentrischen Kontur 311 bilden den ersten Abschnitt 301 und den zweiten Abschnitt 302. Eine Drehung des Exzenterelements 308 um die Drehachse G bewirkt eine Veränderung des auf die Querachse Y projizierten Querabstands zwischen dem ersten Abschnitt 301 und dem zweiten Abschnitt 302. Hierdurch kann das Exzenterelement 308, genauer dessen Kontur 311, über eine Drehung um die Drehachse G innerhalb des Ringspalts S verspannt werden, so dass der erste Abschnitt 301 an den Knochendeckel D und der zweite Abschnitt 302 an den Schädelknochen K radial angepresst werden.

Die beiden Abschnitte 301, 302 ragen zueinander entgegengesetzt nach außen von der Drehachse G ab. Dabei versteht sich, dass die insbesondere in den Fig. 17 bis 19 gezeigte Gestaltgebung der Kontur 311 rein exemplarisch ist. Wesentlich ist allein die Exzentrizität der Kontur 311, d.h. dass die Kontur 311 in Bezug auf die Drehachse G nicht rotationssymmetrisch ist.

Bei der gezeigten Ausführungsform weist das Implantat 300 zudem ein Schaftelement 307 auf, welches entlang der Drehachse G zwischen einem nicht näher bezeichneten ersten Ende und einem nicht näher bezeichneten zweiten Ende gerade längserstreckt ist. Das Exzenterelement 308 ist an dem zweiten Ende des Schaftelements 307 angeordnet und fest mit demselben verbunden. An dem ersten Ende ist ein Werkzeugaufnahmeelement 319 angeordnet und fest mit dem Schaftelement 307 verbunden.

Das Werkzeugaufnahmeelement 319 ist zum Aufbringen eines um die Drehachse G gerichteten Drehmoments eingerichtet und bei der gezeigten Ausführungsform ein Außensechskant.

Das Werkzeugaufnahmeelement 319 bildet bei der gezeigten Ausführungsform ein in Bezug auf die Längsachse X oberes Stirnende des Implantats 300. Das Exzenterelement 308 bildet insoweit ein unteres Stirnende.

Das Implantat 300 weist in Übereinstimmung mit dem Implantat 200 einen plattenförmigen Stützabschnitt 320 auf. Die Funktion des Stützabschnitts 320 ist im Wesentlichen analog zu der des Stützabschnitts 220, so dass zur Vermeidung von Wiederholungen auf das diesbezüglich Offenbarte ausdrücklich verwiesen und Bezug genommen wird.

Das Implantat 300 weist zudem zwei Dornabschnitte 323 auf, die jeweils einends mit einer Dornspitze 324 versehen sind. Andernends sind die Dornabschnitte 323 jeweils fest mit dem Stützabschnitt 320 verbunden. Hinsichtlich ihrer Funktion und Gestaltung sind die Dornabschnitte 323 im Wesentlichen identisch mit dem Dornabschnitt 223 des Implantats 200. Auch insoweit wird ausdrücklich auf das zuvor Gesagte Bezug genommen und verwiesen.

Das Schaftelement 307 ist relativ zu dem Stützabschnitt 320 drehbeweglich an demselben gelagert. Zudem ist das Schaltelement 307 mitsamt des Exzenterelements 308 und des Werkzeugaufnahmeelements 319 in Bezug auf die Längsachse X formschlüssig an dem Stützabschnitt 320 festgelegt. Zu diesem Zweck weist der Stützabschnitt 320 eine nicht näher bezeichnete Durchgangsbohrung auf, die ausgehend von einer Oberseite des Stützabschnitts 320 auf dessen Unterseite erstreckt ist.

Bei der gezeigten Ausführungsform weist das Exzenterelement 308 einen ersten Klingenabschnitt 309 und einen zweiten Klingenabschnitt 310 auf. Der erste Klingenabschnitt 309 bildet gleichsam den ersten Abschnitt 301. Der zweite Klingenabschnitt 310 bildet gleichsam den zweiten Abschnitt 302.

Der erste Klingenabschnitt 309 weist vorliegend eine erste Schneide 312 auf, der zweite Klingenabschnitt 310 weist eine zweite Schneide 313 auf. Die Schneiden 312, 313 sind zueinander entgegengesetzt orientiert.

Bei dem Verspannen des Exzenterelements 308 in dem Ringspalt S dringen die Klingenabschnitte 309, 310 mit ihrer jeweiligen Schneide 312, 313 voran in die jeweils zugeordnete Knochenstruktur ein, d.h. in den Knochendeckel D oder den Schädelknochen K. Durch das Eindringen der Schneiden 312, 313 kann eine verbesserte Fixierung erreicht werden.

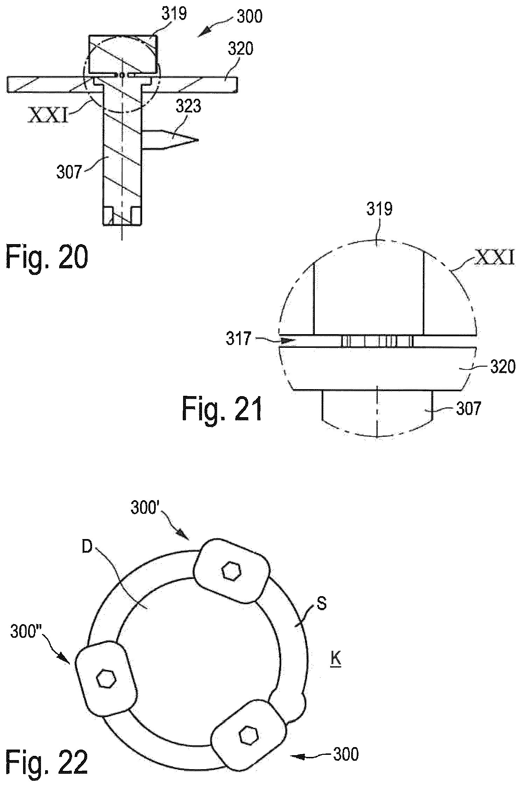

Das Werkzeugaufnahmeelement 319 ist bei der gezeigten Ausführungsform über einen Sollbruchabschnitt 317 (siehe Fig. 21) mit dem Schaftelement 307 verbunden. Der Sollbruchabschnitt 317 weist in Relation zu dem Schaftelement 307 einen verringerten lasttragenden Querschnitt auf. Hierdurch kann das Werkzeugaufnahmeelement 319 nach erfolgter Fixierung über eine zusätzliche Drehmomentbeaufschlagung definiert von dem Schaftelement 307 abgeschert und entfernt werden. Hierdurch wird vermieden, dass das Implantat 300 übermäßig auf die Schädelaußenseite CA aus dem Ringspalt S herausragt. Dies hat wiederum kosmetische und medizinische Vorteile.

Fig. 22 zeigt eine intraoperative Situation unter Verwendung des Implantats 300, wobei wiederum ein aus mehreren Implantaten gebildetes Implantatsystem zum Einsatz kommt. Das Implantatsystem umfasst mehrere baugleiche Implantate 300, 300', 300". Im Übrigen wird zur Vermeidung von Wiederholungen auf die Erläuterungen im Zusammenhang mit den Fig. 10 bis 12 verwiesen. Das zu diesen Erläuterte gilt mutatis mutandis auch hinsichtlich Fig. 22.

Die Fig. 23 bis 29 zeigen unterschiedliche Varianten 300a bis 300f des Implantats 300. Deren Aufbau und Funktionsweise ist im Wesentlichen identisch mit dem Implantat 300. Funktionsgleiche Bauteile und/oder Abschnitte werden nicht gesondert erläutert. Stattdessen wird auf die Offenbarung in Bezug auf das Implantat 300 ausdrücklich verwiesen und Bezug genommen.

Das Implantat 300a unterscheidet sich hinsichtlich der Gestaltung der Werkzeugaufnahme 319a. Die Werkzeugaufnahme 319a ist zur Aufnahme eines Schraubendrehers eingerichtet. Insoweit kann auch von einer Schlitzaufnahme 319a gesprochen werden. Im Unterschied zu der Werkzeugaufnahme 319 ragt die Werkzeugaufnahme 319a in Bezug auf die Längsachse X nicht über den Stützabschnitt hinaus.

Das Implantat 300b nach Fig. 24 unterscheidet sich zum einen durch die Abwesenheit der Dornabschnitte und zum anderen durch die Gestaltung des Exzenterelements 308b. Bei dem Implantat 300b weist der Stützabschnitt 320b zwecks Vorfixierung Bohrlöcher 322b auf. Die Bohrlöcher 322b sind ausgehend von einer Oberseite des Stützabschnitts 320b bis auf dessen Unterseite erstreckt. Zur Vorfixierung kann der Stützabschnitt mit einer durch eine der Bohrungen 322b erstreckte Schraube an dem Knochendeckel D vorfixiert werden.

Das Exzenterelement 308b weist mehrere erste Klingenabschnitte und mehrere zweite Klingenabschnitte auf, die in Bezug auf die Längsachse X ausgehend von dem zweiten Ende des Schaftelements in Richtung des ersten Endes übereinander angeordnet sind. Insoweit kann auch von einer unteren ersten Klinge 309a, einer mittleren ersten Klinge 309a' und einer oberen ersten Klinge 309a" gesprochen werden. Dementsprechend weist das Exzenterelement 308b eine untere zweite Klinge 310a, eine mittlere zweite Klinge 310a' und eine obere zweite Klinge 310a" auf. Es versteht sich, dass anstelle der vorliegend drei übereinander angeordneten Klingenpaare mehr oder weniger Klingenpaare vorhanden sein können.

Bei der Variante nach Fig. 25 ist lediglich ein Bauteil des Implantats 300c, nämlich dessen Schaftelement 307c, dargestellt. Um zu vermeiden, dass das Schaftelement 307c sich mitsamt dem Exzenterelement unbeabsichtigt entgegen der Fixierungsrichtung um die Drehachse G zurückdreht, weist das Schaftelement 307c im Bereich seines ersten Endes ein Gewinde 325c auf. Das Gewinde 325c ist auf zeichnerisch nicht näher gezeigte Weise mit einem in den Stützabschnitt eingebrachten Gegengewinde verschraubt. Das Gegengewinde kann beispielsweise als Stoppmutter oder dergleichen ausgeführt sein.

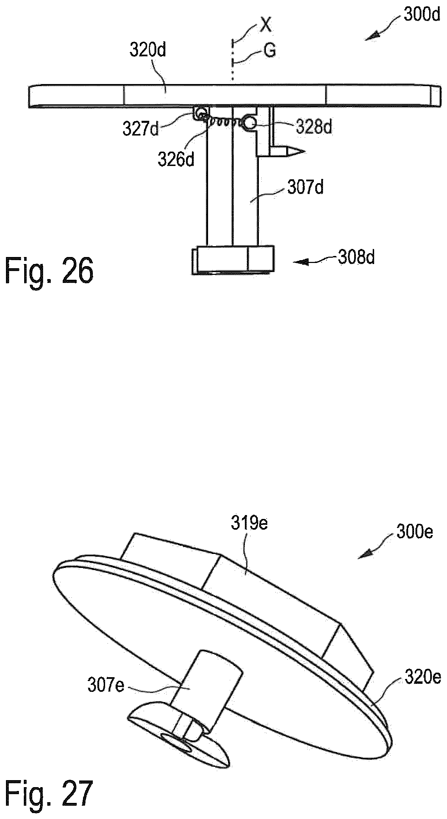

Bei der Variante nach Fig. 26 weist das Implantat 300d anstelle einer Werkzeugaufnahme zur Aufbringung eines Drehmoments ein Federelement 326d auf. Das Federelement 326d ist einends an einem ersten Befestigungsabschnitt 327d festgelegt und andernends an einem zweiten Befestigungsabschnitt 328d festgelegt. Der erste Befestigungsabschnitt 327d ist dem Stützabschnitt 320d zugeordnet und fest mit demselben verbunden. Der zweite Befestigungsabschnitt 328d ist dem Schaftelement 307d zugeordnet und fest mit demselben verbunden. In der anhand Fig. 26 gezeigten Konfiguration ist das Federelement 326d vorgespannt und bewirkt ein um die Längsachse X gerichtetes Drehmoment auf das Schaftelement 307d. Dabei ist eine zeichnerisch nicht näher gezeigte Arretiereinrichtung vorhanden, mittels derer das Schaftelement 307d in der vorgespannten Situation gehalten ist. Die Arretiereinrichtung ist zwischen einem ersten Zustand und einem zweiten Zustand überführbar. In dem ersten Zustand der Arretiereinrichtung ist das Schaftelement 307d relativ zu dem Stützabschnitt 302d rotatorisch festgelegt. Im dem zweiten Zustand ist die Rotationsbeweglichkeit freigegeben. Nach Freigabe der Rotationsbeweglichkeit bewirkt das Federelement 326d eine Rotation des Schaftelements 307d - und damit auch des Exzenterelements 308d - um die Drehachse G. Hierdurch wird das Exzenterelement 308d in dem Ringspalt S verspannt und/oder verkeilt.

Bei der Variante nach Fig. 27 weist das Implantat 300e im Unterschied zu dem Implantat 300 und den vorhergehenden Varianten 300a bis 300d eine drehmomentfeste Verbindung zwischen dem Stützabschnitt 320e und dem Schaftelement 307e auf. Folglich rotieren der Stützabschnitt 320e und das Schaftelement 307e stets gemeinsam um die Drehachse G. Der Stützabschnitt 320e und das Schaftelement 307e können kraft-, form- und/oder stoffschlüssig miteinander verbunden sein. Zudem ist eine einstückig zusammenhängende Ausbildung denkbar. Die Werkzeugaufnahme 319e ist bei dem Implantat 300e fest auf einer Oberseite des Stützabschnitts 320e angeordnet.

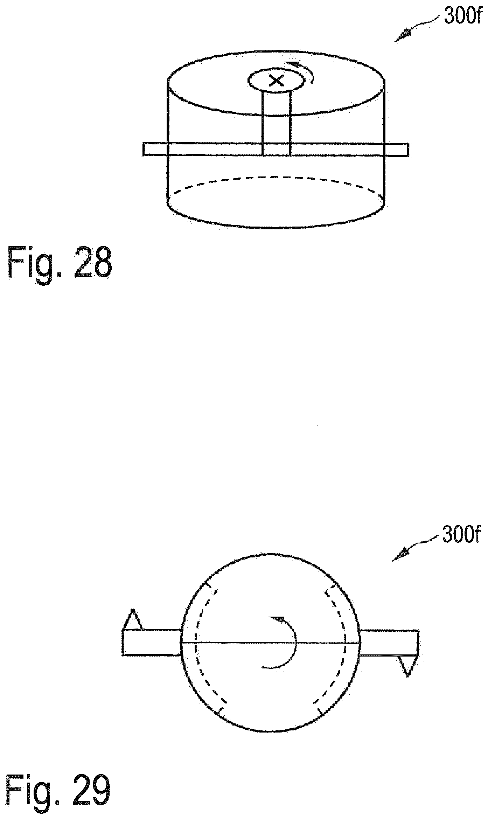

Bei der Variante nach den Fig. 28 und 29 weist das Implantat 307f ein entsprechendes Bohrlochdesign auf, welches an das Prinzip einer Hohlraumdose angelehnt ist.

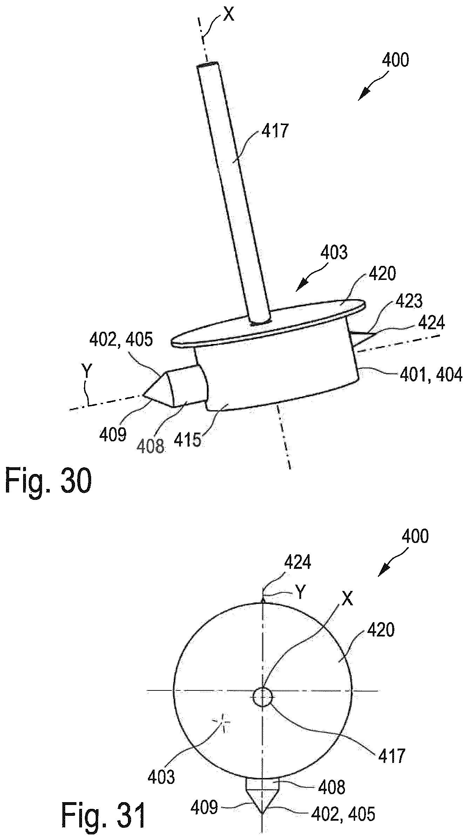

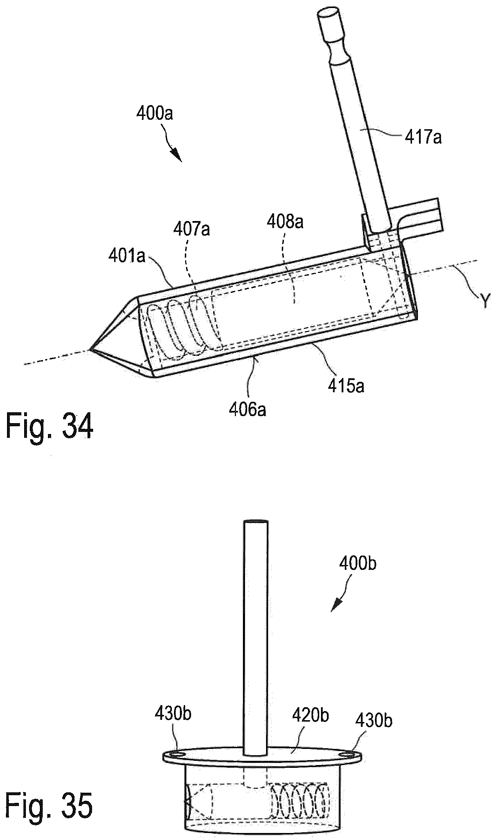

Die Fig. 30 bis 35 zeigen eine weitere Ausführungsform eines erfindungsgemäßen Implantats 400, wobei die Fig. 34 und 35 Varianten dieser Ausführungsform betreffen.

Gemäß den Fig. 30 bis 32 weist das Implantat 400 eine Längsachse X, eine Querachse Y, einen ersten Abschnitt 401, einen zweiten Abschnitt 402 und einen Mechanismus 403 auf.

Hinsichtlich der Orientierung der Längsachse X und der Querachse Y in Bezug auf die Axialrichtung A und die Radialrichtung R der kranialen Öffnung B gilt das zu den vorhergehenden Ausführungsformen Gesagte.

Der erste Abschnitt 401 ist wiederum zur Kraftübertragung auf den Knochendeckel D eingerichtet. Der zweite Abschnitt 402 ist dementsprechend zur Kraftübertragung auf den Schädelknochen K eingerichtet. Der Mechanismus 403 ist auf noch näher beschriebene Weise mit dem ersten Abschnitt 401 und dem zweiten Abschnitt 402 wirkverbunden und erlaubt eine Veränderung des Querabstands zwischen diesen beiden Abschnitten.

Im Speziellen weist der Mechanismus ein Druckelement 408 und ein Federelement 407 auf.

Das Druckelement 408 ist entlang der Querachse Y zwischen einem ersten Stirnende 409 und einem zweiten Stirnende 410 längserstreckt. Zudem ist das Druckelement 408 entlang der Querachse Y beweglich gelagert.

Das Federelement 407 ist einends kraftübertragend an dem zweiten Abschnitt 410 des Druckelements abgestützt. Andernends ist das Federelement 407 wenigstens mittelbar mit dem ersten Abschnitt 401 wirkverbunden. Bei der gezeigten Ausführungsform bildet das Druckelement 408, genauer: dessen erstes Stirnende 409, den zweiten Abschnitt 402 und/oder fungiert als zweite Kontaktfläche 405.

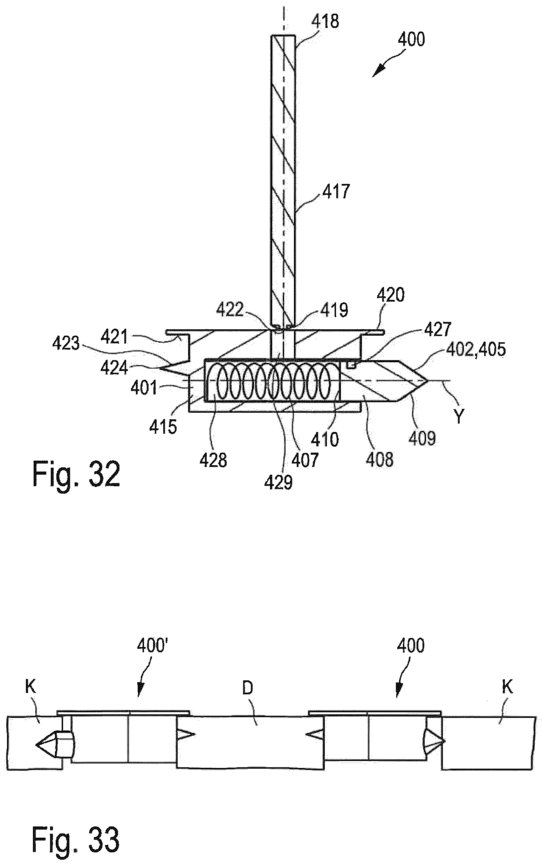

Das Federelement 407 dient einer Vorspannung des Druckelements 408 entlang der Querachse Y. Das Druckelement 408 ist durch die besagte Vorspannung entlang der Querachse Y und in Bezug auf die Radialrichtung R nach außen gedrückt und auf diese Weise an den Schädelknochen K anpressbar (siehe Fig. 33).

In den Fig. 30 bis 32 ist eine äußere Endlage des Druckelements 408 gezeigt. In dieser äußeren Endlage ist das Druckelement 408 unter Einwirkung des Federelements 407 entlang der Querachse Y kraftbeaufschlagt nach außen verlagert. In einer in den Figuren nicht gezeigten inneren Endlage ist das Druckelement 408 entlang der Querachse Y nach innen verlagert, das Federelement 407 ist entlang seiner Axialrichtung zusammengedrückt und auf diese Weise vorgespannt.

Zur Arretierung der besagten Vorspannung ist vorliegend ein Arretierelement 417 vorhanden. Das Arretierelement 417 ist relativ zu dem Druckelement 408 zwischen einer Arretierposition und einer Freigabeposition verlagerbar. In der Arretierposition ist das Druckelement 408 mittels des Arretierelements 417 entgegen der Federkraft des (vorgespannten) Federelements 407 in Bezug auf die Querachse Y festgelegt. In der Freigabeposition ist die Beweglichkeit des Druckelements 408 freigegeben, so dass das Federelement 407 das Druckelement 408 in Richtung der äußeren Endlage verlagern kann.

Bei der gezeigten Ausführungsform ist das Arretierelement 417 entlang der Längsachse X zwischen einem ersten Stirnende 418 und einem zweiten Stirnende 419 längserstreckt. Das zweite Stirnende 419 weist einen Formschlussabschnitt 422 auf, der in der Arretierposition entlang der Querachse Y formschlüssig mit einem komplementären Formschlussabschnitt 427 des Druckelements 408 zusammenwirkt. Bei der gezeigten Ausführungsform ist der Formschlussabschnitt 422 ein entlang der Längsachse X von dem zweiten Stirnende 419 abragender Vorsprung. Dementsprechend ist der komplementäre Formschlussabschnitt 427 eine entlang der Längsachse X in das Druckelement 408 eingesenkte Einsenkung. In der Arretierposition ist der Vorsprung in die Einsenkung eingeführt, so dass das Druckelement 408 in Querrichtung formschlüssig an dem Arretierelement 417 gehalten ist.

Zur Verlagerung in die Freigabeposition wird das Arretierelement 417 axial - und in Bezug auf die Zeichenebene der Fig. 32 - nach oben verlagert. Hierdurch wird der Formschlussabschnitt 422 aus dem komplementären Formschlussabschnitt 427 herausgezogen. Die gezeigte Ausführungsform sieht vor, dass das Arretierelement 417 nach einer Verlagerung in die Freigabeposition vollständig entfernt werden kann (siehe Fig. 33). Dabei ist das Arretierelement 417 zur manuellen Betätigung vorgesehen. Zur Verlagerung in Richtung der Freigabeposition kann der Operateur das Arretierelement 417 im Bereich des ersten Stirnendes 418 beispielsweise zwischen dem Daumen und dem Zeigefinger einer Hand greifen.

Zwecks Vorfixierung weist das Implantat 400 einen Dornabschnitt 423 mit einer Dornspitze 424 auf. Die Funktion und Gestaltung des Dornabschnitts 423 ist im Wesentlichen identisch zu den vorhergehenden Ausführungsformen, so dass das dort Gesagte mutatis mutandis auch vorliegend gilt.

Zum Abstützen auf der Schädelaußenseite CA ist zudem ein Stützabschnitt 420 vorhanden. Dessen Unterseite 421 wird zur Positionierung des Implantats 400 in dem Ringspalt S zum einen auf der Außenseite DF des Knochendeckels D und zum anderen auf der Außenseite KF des Schädelknochens abgestützt. Im Übrigen wird auf das zu den Stützabschnitten 220, 320 der vorhergehenden Ausführungsformen Gesagte verwiesen und ausdrücklich Bezug genommen.

Weiter weist das Implantat 400 bei der gezeigten Ausführungsform ein Gehäuse 415 auf. Das Gehäuse 415 dient vorliegend insbesondere einer Aufnahme und/oder Lagerung des Federelements 407, des Druckelements 408 und des Arretierelements 417. Zudem ist der erste Abschnitt 401 auf einer dem ersten Stirnende 409 entlang der Querachse Y diametral gegenüberliegenden Seite des Gehäuses 415 angeordnet und/oder ausgebildet. Der besagte Abschnitt des Gehäuses 415 fungiert insoweit als erste Kontaktfläche 404.

Das Gehäuse 415 weist vorliegend eine erste Aufnahmeaussparung 428 und eine zweite Aufnahmeaussparung 429 auf.

Die erste Aufnahmeaussparung 428 ist entlang der Querachse Y längserstreckt und dient der Aufnahme des Federelements 407 und des Druckelements 408. Bei der gezeigten Ausführungsform weist die erste Aufnahmeaussparung 428 einen zylindrischen, genauer: kreiszylindrischen, Querschnitt auf. Die Formgebung des Druckelements 408 ist hierauf abgestimmt und dementsprechend komplementär zylindrisch, genauer: kreiszylindrisch. Die erste Aufnahmeaussparung 428 ist einends geschlossen und andernends offen. Das Druckelement 408 ragt durch die andernends der ersten Aufnahmeaussparung 428 vorhandene und nicht näher bezeichnete Öffnung. Das Druckelement 408 ist entlang der Querachse Y gleitbeweglich in der ersten Aufnahmeaussparung 428 geführt. Das Federelement 407 ist einends der ersten Aufnahmeaussparung 428 an einer nicht näher bezeichneten Wandung des Gehäuses 415 abgestützt. Die besagte Wandung bildet gleichsam den ersten Abschnitt 401.

Die zweite Aufnahmeaussparung 429 ist ausgehend von einer Oberseite des Stützabschnitts 420 entlang der Längsachse X bis in die erste Aufnahmeaussparung 428 hineinerstreckt. Die zweite Aufnahmeaussparung 429 dient der Aufnahme des zweiten Endes 419 des Arretierelements 417. Das Arretierelement 417 ist insoweit entlang der Längsachse X gleitbeweglich in der zweiten Aufnahmeaussparung 429 geführt.

Die erste Aufnahmeaussparung 428 kann auch als Radialbohrung bezeichnet werden. Die zweite Aufnahmeaussparung 429 kann auch als Axialbohrung bezeichnet werden.

Bei der gezeigten Ausführungsform sind der Dornabschnitt 423 und der Stützabschnitt 420 einstückig an dem Gehäuse 415 ausgebildet. Es versteht sich, dass die besagten Abschnitte stattdessen auch als separate Bauteile ausgebildet sein können.

Weiter weist das Gehäuse 415 bei der gezeigten Ausführungsform eine kreiszylindrische Grundform auf, wobei deren Axialrichtung parallel zur Längsachse X erstreckt ist. Infolge der kreiszylindrischen Gestaltung des Gehäuses 415 ist der erste Abschnitt 401 dementsprechend konvex gekrümmt. Hierdurch wird ein verbesserter Kontakt mit dem Außenumfang DA des Knochendeckels D ermöglicht.

Fig. 33 zeigt eine intraoperative Situation unter Verwendung des Implantats 400, in welcher der Knochendeckel D bereits vollständig fixiert ist. Dies unter Zuhilfenahme wenigstens eines weiteren baugleichen Implantats 400'. Zur Vermeidung von Wiederholungen wird auf das zu Fig. 12 Gesagte verwiesen.

Die Fig. 34 und 35 zeigen Varianten des Implantats 400 nach den Fig. 30 bis 33. Zur Vermeidung von Wiederholungen werden nachfolgend lediglich wesentliche Unterschiede der Implantate 400a und 400b gegenüber dem Implantat 400 erläutert. Im Übrigen wird ausdrücklich auf das bereits zu dem Implantat 400 Gesagte Bezug genommen und verwiesen.

Bei der Variante nach Fig. 34 ist das Implantat 400a zur Vorfixierung in einer radial in den Außenumfang DA des Knochendeckels D erstreckten Bohrung H eingerichtet (vgl. Fig. 4). Der erste Abschnitt 401a weist dementsprechend eine Fügefläche 406a auf, die zum Einfügen in das besagte Bohrloch H eingerichtet ist. Insoweit kann von einem Gehäuse 415a gesprochen werden, dessen zylindrischer Außenumfang die Fügefläche 406a und gleichsam den ersten Abschnitt 401a bildet. Das Gehäuse 415a weist wiederum nicht näher bezeichnete Aufnahmeaussparungen zur Aufnahme des Federelements 407a und des Druckelements 408a einerseits und des Arretierelements 417a andererseits auf.

Im Übrigen nimmt das Arretierelement 417a in Fig. 34 seine Arretierposition ein. Das Druckelement 408a ist dementsprechend in seiner inneren Endlage entgegen der Federkraft des Federelements 407a in Bezug auf die Querachse Y festgelegt.

Bei der Variante nach Fig. 35 weist das Implantat 400b keinen Dornabschnitt auf. Stattdessen sind zur Vorfixierung Durchgangsbohrungen 430b in den Stützabschnitt 420b eingebracht. Zur Vorfixierung wird das Implantat 400b mit dem Knochendeckel D verschraubt. Die Durchgangsbohrungen 430b dienen der Aufnahme der hierfür notwendigen Schraube.

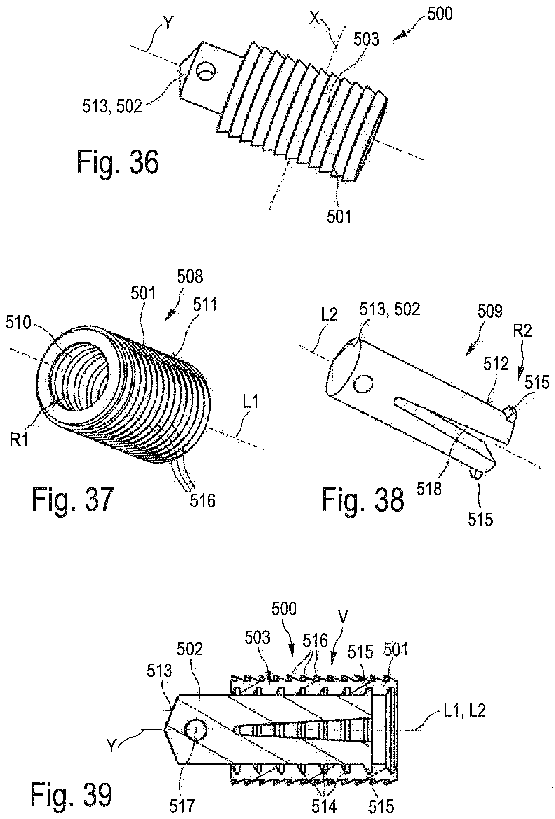

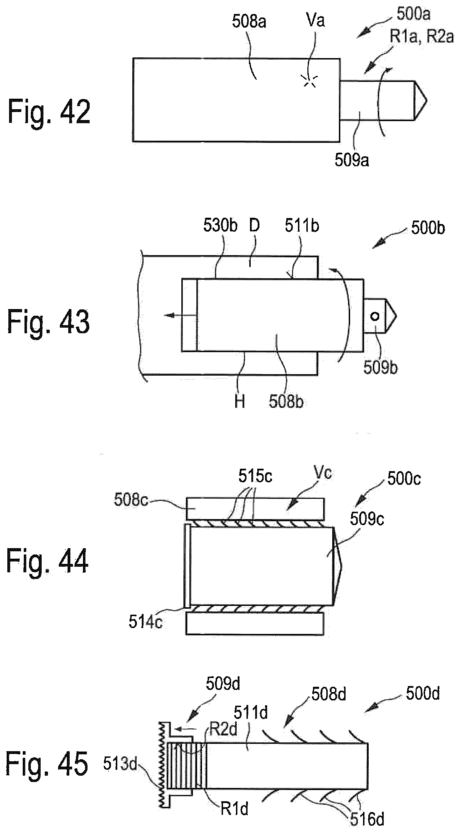

Die Fig. 36 bis 45 zeigen eine weitere Ausführungsform eines erfindungsgemäßes Implantat 500, wobei die Fig. 42 bis 45 Varianten derselben betreffen.

Mit Bezug auf die Fig. 36 bis 39 weist das Implantat 500 eine Längsachse X, eine Querachse Y, einen ersten Abschnitt 501, einen zweiten Abschnitt 502 und einen Mechanismus 503 auf.

Hinsichtlich der Orientierung der Längsachse X und der Querachse Y in Bezug auf die Axialrichtung A und die Radialrichtung R der kranialen Öffnung B gilt das zu den vorhergehenden Ausführungsformen Gesagte sinngemäß.

Der erste Abschnitt 501 ist zur Kraftübertragung auf den Knochendeckel D eingerichtet. Der zweite Abschnitt 502 ist zur Kraftübertragung auf den die kraniale Öffnung B umrandenden Schädelknochen K eingerichtet.

Der mit den beiden Abschnitten 501, 502 wirkverbundene Mechanismus 503 ist in prinzipieller Übereinstimmung mit den vorhergehenden Ausführungsformen zur Vergrößerung des Querabstands zwischen den beiden Abschnitten 501, 502 eingerichtet. Insoweit sind die beiden Abschnitte 501, 502 auch bei dieser Ausführungsform mittels des Mechanismus 503 einerseits an den Knochendeckel D und andererseits an den Schädelknochen K anpressbar.

Bei dieser Ausführungsform weist der Mechanismus 503 ein erstes Zylinderelement 508 (Fig. 37) und ein zweites Zylinderelement 509 auf (Fig. 38). Das erste Zylinderelement 508 weist eine erste Längsachse L1 auf und das zweite Zylinderelement 509 weist eine zweite Längsachse L2 auf. Die beiden Längsachsen L1, L2 sind zueinander koaxial sowie entlang, im Speziellen ebenfalls koaxial zu, der Querachse Y orientiert.

Das erste Zylinderelement 508 weist eine profilierte erste Zylindermantelfläche 510 und eine erste Fläche 511 auf. Die erste Fläche 511 bildet den und/oder fungiert als erster Abschnitt 501.

Das zweite Zylinderelement 509 weist eine profilierte zweite Zylindermantelfläche 512 und eine zweite Fläche 513 auf. Die zweite Fläche 513 fungiert als und/oder bildet den zweiten Abschnitt 502.

Bei der gezeigten Ausführungsform ist die zweite Fläche 513 eine Stirnendfläche des zweiten Zylinderelements 509. Diese ist zur radialen Anlage an dem Innenumfang KI des Schädelknochens K eingerichtet. Die erste Fläche 511 ist eine Außenmantelfläche des ersten Zylinderelements 508 und zum Einfügen in eine radial in den Außenumfang DA des Knochendeckels D erstreckte Bohrung H eingerichtet (vgl. Fig. 4).

Die profilierte erste Zylindermantelfläche 510 ist bei der gezeigten Ausführungsform eine Innenmantelfläche des ersten Zylinderelements 508. Die profilierte zweite Zylindermantelfläche 512 ist bei der gezeigten Ausführungsform eine Außenmantelfläche des zweiten Zylinderelements 509. Folglich ist das erste Zylinderelement 508 bei der gezeigten Ausführungsform ein Hohlzylinder, der auch als Buchse bezeichnet werden kann; das zweite Zylinderelement 509 kann dementsprechend als Bolzen, Stift oder dergleichen bezeichnet werden. Bei einer weiteren Ausführungsform ist stattdessen eine umgekehrte Zuordnung vorgesehen, so dass das erste Zylinderelement als Bolzen und das zweite Zylinderelement als Buchse gestaltet ist.

In gebrauchsfertig assembliertem Zustand (Fig. 36, 39) sind das erste Zylinderelement 508 und das zweite Zylinderelement 509 miteinander axial zusammengesteckt. Genauer ist das zweite Zylinderelement 509 in das erste Zylinderelement 508 axial eingesteckt. Hierbei bilden die profilierte erste Zylindermantelfläche 510 und die profilierte zweite Zylindermantelfläche 512 eine entlang der Querachse Y form- und/oder kraftschlüssige Verbindung V aus (Fig. 39). Die besagte Verbindung V kann in unterschiedlichen axialen Relativpositionen der beiden Zylinderelemente 508, 509 etabliert werden, wodurch letztlich der Querabstand zwischen dem ersten Abschnitt 501 und dem zweiten Abschnitt 502 veränderlich ist.

Bei der gezeigten Ausführungsform ist die besagte Verbindung V eine Rastverbindung. Dementsprechend sind die erste Zylindermantelfläche 510 und die zweite Zylindermantelfläche 512 jeweils mittels einer Rastgeometrie R1, R2 profiliert, die auch als erste Rastgeometrie R1 und zweite Rastgeometrie R2 bezeichnet werden können.

Die erste Rastgeometrie R1 weist bei der gezeigten Ausführungsform mehrere entlang der ersten Längsachse L1 voneinander beabstandete und jeweils in Radialrichtung des ersten Zylinderelements 508 in dessen Innenmantelfläche eingesenkte Umfangsrillen 514 auf (Fig. 39). Die zweite Rastgeometrie R2 des zweiten Zylinderelements 509 weist bei der gezeigten Ausführungsform zwei in Umfangsrichtung des zweiten Zylinderelements 509 um 180° versetzt angeordnete Rastvorsprünge 515 auf. Diese sind komplementär zu den Umfangsrillen 514 geformt. Vorliegend sind die Rastvorsprünge 515 an einem der zweiten Fläche 513 abgewandten Ende des zweiten Zylinderelements 509 angeordnet.

Wie anhand Fig. 39 gezeigt ist, sind die beiden Rastgeometrien R1, R2 vorliegend derart gestaltet, dass die Rastverbindung V einseitig entlang der Querachse Y überrastbar ist. Folglich kann der Querabstand bei der gezeigten Ausführungsform lediglich vergrößert, jedoch nicht verkleinert werden. Mit anderen Worten ausgedrückt, können das erste Zylinderelement 508 und das zweite Zylinderelement 509 auseinandergezogen, aber nicht zusammengedrückt werden.

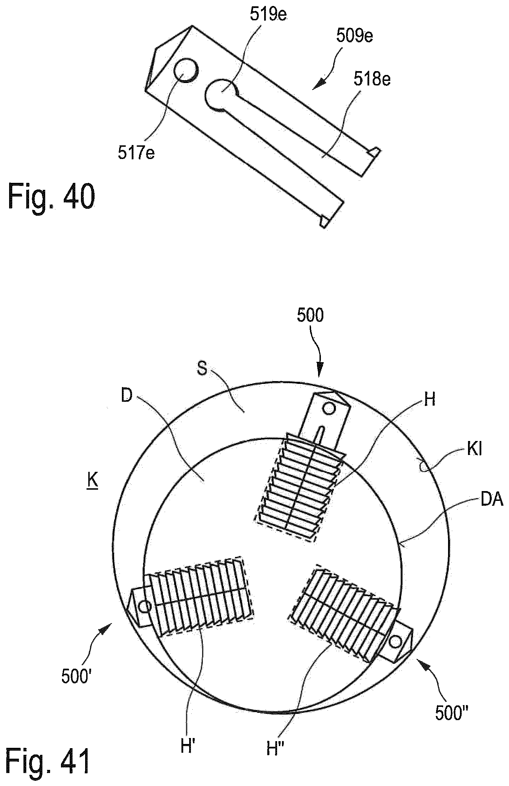

Fig. 41 zeigt eine intraoperative Situation, in welcher der Knochendeckel D unter Verwendung des Implantats 500 fixiert ist. Zur Vorfixierung wird das Implantat 500 mit dem ersten Zylinderelement 508 voran zunächst in die Bohrung H eingesetzt. Bei der gezeigten Ausführungsform weist das erste Zylinderelement 508 an seiner Außenmantelfläche eine nicht näher bezeichnete Profilierung mit mehreren entlang der ersten Längsachse L1 beabstandet angeordneten und jeweils umlaufend erstreckten Vorsprüngen 516 auf. Diese Vorsprünge dienen einer verbesserten Vorfixierung und bilden eine Art Verzahnung. Nach erfolgter Vorfixierung wird der Knochendeckel D mitsamt dem Implantat 500 in die Öffnung B eingesetzt. Durch ein einseitiges Überrasten der Rastverbindung V wird der zweite Abschnitt 502 - in diesem Fall die Stirnendfläche 513 des zweiten Zylinderelements 509 - zur Anlage an den Innenumfang KI des Schädelknochens K gebracht. Die hierfür erforderliche Zugkraft entlang der Querachse Y wird vorliegend über eine Zugöse 517 aufgebracht. Die Zugöse 517 ist vorliegend eine im Bereich des Stirnendes 513 angeordnete Querbohrung. Zur Zugkraftbeaufschlagung des zweiten Zylinderelements 509 kann beispielsweise ein hierfür geeignetes Werkzeug in die Zugöse 517 eingebracht werden.

Um das Überrasten der Rastverbindung V zu erleichtern, weist das zweite Zylinderelement 509 bei der gezeigten Ausführungsform einen Längsschlitz 518 auf. Der Längsschlitz 518 erstreckt sich einends axial in das zweite Zylinderelement 509 und ermöglicht eine radiale Federbeweglichkeit der Rastvorsprünge 515. Dabei ist anhand Fig. 40 eine geringfügige Abwandlung gezeigt, bei welcher ein zweites Zylinderelement 509e mit einem modifizierten Längsschlitz 518e versehen ist. Der Längsschlitz 518e weist an seinem geschlossenen Ende eine Aufweitung 519e auf. Die Aufweitung 519e ist im Bereich der Zugöse 517e angeordnet und ähnlich einer Durchgangsbohrung parallel zu dieser erstreckt. Die Aufweitung 519e unterstützt eine anforderungsgerechte federelastische Beweglichkeit der Rastvorsprünge.

Weiter in Bezug auf Fig. 41 ist gezeigt, dass der Knochendeckel D wiederum mittels mehrerer Implantate 500, 500', 500" fixiert ist. Eine solche Fixierung mittels mehrerer, insbesondere baugleicher, Implantate ist vorteilhaft, aber nicht zwingend. Prinzipiell ist es denkbar, dass der Knochendeckel D lediglich unter Verwendung eines einzigen Implantats fixiert wird.

Die Fig. 42 bis 45 zeigen Varianten des Implantats 500. Die Funktion und Gestaltung der gezeigten Varianten ist im Wesentlichen identisch zu dem Implantat 500. Nachfolgend wird zur Vermeidung von Wiederholungen lediglich auf wesentliche Unterschiede eingegangen. Funktionsbaugleiche Teile und/oder Abschnitte werden nicht gesondert erläutert. Stattdessen wird ausdrücklich auf das zu dem Implantat 500 Gesagte verwiesen und Bezug genommen.

Bei der Variante nach Fig. 42 weist das Implantat 500a anstelle der Rastverbindung V eine Schraubverbindung Va auf. D.h. das erste Zylinderelement 508a und das zweite Zylinderelement 509a sind entlang der Querachse Y gewindebeweglich miteinander verschraubt. Zu diesem Zweck weist das erste Zylinderelement 508a eine erste Gewindegeometrie R1a auf. Das zweite Zylinderelement 509a weist eine komplementäre zweite Gewindegeometrie R2a auf. Die erste Gewindegeometrie R1a ist vorliegend ein Innengewinde. Die zweite Gewindegeometrie R2a ist ein Außengewinde.

Bei der Variante nach Fig. 43 ist vorgesehen, dass das Implantat 500b in die Bohrung H eingeschraubt wird. Zu diesem Zweck weist die Außenmantelfläche 511b ein zeichnerisch nicht im Detail gezeigtes Außengewinde 530b auf. Das Außengewinde 530b ist vorzugsweise ein selbstschneidendes und/oder selbstbohrendes Gewinde.

Bei der Variante nach Fig. 44 weist das Implantat 500c eine abgewandelte Rastverbindung Vc auf. Die Rastverbindung Vc ist zwischen einem stirnendseitig an dem zweiten Zylinderelement 509c angeordneten Radialbund 514c und an der Innenmantelfläche des ersten Zylinderelements 508c angeordneten Federelementen 515c ausgebildet. Die Federelemente 515c sind in Radialrichtung der Zylinderelemente 508c, 509c nach außen federbeweglich nachgiebig.

Bei der Variante nach Fig. 45 ist - vereinfacht ausgedrückt - eine umgekehrte Zuordnung der Buchsengeometrie einerseits und der Bolzengeometrie andererseits auf das erste Zylinderelement 508d und das zweite Zylinderelement 509d vorgesehen. Dementsprechend weist das Implantat 500d ein bolzenförmiges erstes Zylinderelement 508d und ein buchsenförmiges zweites Zylinderelement 509d auf. Das erste Zylinderelement 508d ist zum Einfügen in eine Bohrung H des Knochendeckels D eingerichtet und weist auf seiner Außenmantelfläche 511d mehrere Verankerungselemente 516d auf. Das zweite Zylinderelement 509d ist einends auf das erste Zylinderelement 508d aufgesteckt und mit demselben verrastet. Zu diesem Zweck weist die Außenmantelfläche 511d abschnittsweise eine erste Rastprofilierung R1d auf. Eine nicht näher bezeichnete Innenmantelfläche des zweiten Zylinderelements 509d weist eine hierzu komplementäre zweite Rastprofilierung R2d auf. Die Stirnfläche 513d des zweiten Zylinderelements 509d ist mit einer nicht näher bezeichneten Verzahnung versehen. Diese ermöglicht einen verbesserten Kontakt mit dem Innenumfang KI des Schädelknochens.

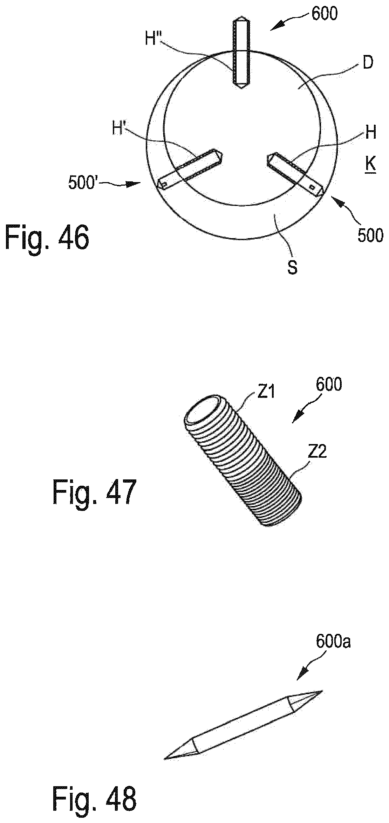

Fig. 46 zeigt eine weitere intraoperative Situation, in welcher der Knochendeckel D unter Verwendung eines ersten Implantats 500, eines baugleichen weiteren Implantats 500' und eines Pins 600 fixiert ist. Der Pin 600 ist im Detail in Fig. 47 gezeigt und weist an seiner Außenmantelfläche eine erste Zahnung Z1 und eine zweite Zahnung Z2 auf. Die erste Zahnung Z1 dient einer verbesserten Fixierung des Pins in der Bohrung H" des Knochendeckels D (siehe Fig. 46). Die zweite Zahnung Z2 dient einer verbesserten Verankerung des Pins 600 in einer nicht näher bezeichneten, in den umliegenden Schädelknochen K eingebrachten Bohrung.

Fig. 48 zeigt einen alternativ ausgeführten Pin 600a. Der Pin 600a ist an seinen axial gegenüberliegenden Stirnenden angespitzt. Die angespitzten Stirnenden ermöglichen, dass der Pin 600a zum einen in den Außenumfang DA des Knochendeckels D und zum anderen in den Innenumfang KI des Schädelknochens eingedrückt werden kann. Hierdurch kann im Unterschied zu dem Pin 600 auf das Einbringen von Bohrungen in den Schädelknochen K und/oder den Knochendeckel D verzichtet werden.

## Patentansprüche

1. Implantat (100, 200, 300, 400, 500) zur Fixierung eines kranialen Knochendeckels (D) in einer kranialen Öffnung (B), aufweisend
eine Längsachse (X), welche entlang einer Axialrichtung (A) der kranialen Öffnung (B) ausgerichtet ist,
eine Querachse (Y), welche entlang einer Radialrichtung (R) der kranialen Öffnung (B) ausgerichtet ist,
einen ersten Abschnitt (101, 201, 301, 401, 501), welcher zur Kraftübertragung auf den Knochendeckel (D) eingerichtet ist,
einen zweiten Abschnitt (102, 202, 302, 402, 502), welcher zur Kraftübertragung auf einen die kraniale Öffnung (B) umrandenden Schädelknochen (K) eingerichtet ist,
wobei der erste Abschnitt (101, 201, 301, 401, 501) und/oder der zweite Abschnitt (102, 202, 302, 402, 502) zur Anordnung in einem zwischen einem Außenumfang (DA) des Knochendeckels (D) und einem Innenumfang (KI) des Schädelknochens (K) ausgebildeten Ringspalt (S) eingerichtet ist,
und aufweisend einen mit dem ersten Abschnitt (101, 201, 301, 401, 501) und dem zweiten Abschnitt (102, 202, 302, 402, 502) wirkverbundenen Mechanismus (103, 203, 303, 403, 503), mittels dessen ein auf die Querachse (Y) projizierter Querabstand (Q) zwischen dem ersten Abschnitt (101, 201, 301, 401, 501) und dem zweiten Abschnitt (102, 202, 302, 402, 502) wenigstens vergrößerbar ist, wodurch der erste Abschnitt (101, 201, 301, 401, 501) in Radialrichtung (R) nach innen an den Knochendeckel (D) und der zweite Abschnitt (102, 202, 302, 402, 502) in Radialrichtung (R) nach außen an den Schädelknochen (K) anpressbar sind,
wobei ein Stützabschnitt (220, 320, 420) vorhanden ist, welcher entlang der Längsachse (X) oberhalb des ersten Abschnitts (201, 301, 401) und des zweiten Abschnitts (202, 302, 402) angeordnet ist und eine Unterseite (221, 321, 421) aufweist, welche zum Abstützen auf einer Außenseite (KF) des Schädelknochens (K) und einer Außenseite (DF) des Knochendeckels (D) eingerichtet ist,
**dadurch gekennzeichnet, dass** der Stützabschnitt (220) mittels einer lösbaren Fügeverbindung, insbesondere einem Bajonettverschluss, mit dem Implantat (200) im Übrigen verbunden ist.

2. Implantat (100, 200, 400, 500) nach Anspruch 1, **dadurch gekennzeichnet, dass** der Mechanismus (103, 203, 403, 503) zur translatorischen Verlagerung des ersten Abschnitts (101, 201, 401, 501) und/oder des zweiten Abschnitts (102, 202, 402, 502) entlang der Querachse (Y) eingerichtet ist.

3. Implantat (300) nach Anspruch 1 oder 2, **dadurch gekennzeichnet, dass** der Mechanismus (303) zur rotatorischen Verlagerung des ersten Abschnitts (301) und/oder des zweiten Abschnitts (302) um die Längsachse (X) eingerichtet ist.

4. Implantat (100, 200, 300, 400, 500) nach einem der vorhergehenden Ansprüche, **dadurch gekennzeichnet, dass** der erste Abschnitt (101, 201, 301, 401, 501) eine erste Kontaktfläche aufweist, welche zur radialen Anlage an dem Außenumfang (DA) des Knochendeckels (D) eingerichtet ist, und/oder dass der zweite Abschnitt (102, 202, 302, 402, 502) eine zweite Kontaktfläche aufweist, welche zur radialen Anlage an dem Innenumfang (KI) des Schädelknochens eingerichtet ist.

5. Implantat (100, 500) nach einem der vorhergehenden Ansprüche, **dadurch gekennzeichnet, dass** der erste Abschnitt (101, 501) eine Fügefläche (106, 511) aufweist, welche zum Einfügen in eine radial in den Außenumfang (DA) des Knochendeckels (D) erstreckte Bohrung (H) eingerichtet ist.

6. Implantat (200, 300, 400) nach einem der vorhergehenden Ansprüche, **dadurch gekennzeichnet, dass** wenigstens ein Dornabschnitt (223, 323, 423) vorhanden ist, welcher entlang der Querachse (Y) längserstreckt ist und einends eine über den ersten Abschnitt (201, 301, 401) hinausragende Dornspitze (224, 324, 424) aufweist, welche zum radialen Einstechen in den Außenumfang (DA) des Knochendeckels (D) eingerichtet ist.

7. Implantat (200) nach einem der vorhergehenden Ansprüche, **dadurch gekennzeichnet, dass** der Mechanismus (203) wenigstens ein Spannelement (208) und ein Kegelelement (207) aufweist, wobei das Spannelement (208) entlang der Querachse (Y) beweglich gelagert ist und eine Innenkonusfläche (210) sowie eine den ersten Abschnitt (201) oder den zweiten Abschnitt (202) bildende Stirnfläche (211) aufweist, wobei das Kegelelement (207) entlang der Längsachse (X) beweglich gelagert ist und eine mit der Innenkonusfläche (210) zusammenwirkende Außenkonusfläche (214) aufweist, und wobei das Spannelement (208) mittels einer Bewegung des Kegelelements (207) entlang der Querachse (Y) verlagerbar ist, insbesondere wobei der Mechanismus (203) ein weiteres Spannelement (209) aufweist, welches mittels der Bewegung des Kegelelements (207) entlang der Querachse (Y) entgegengesetzt verlagerbar ist, und/oder wobei das Kegelelement (207) ein Gewinde (216) aufweist, welches entlang der Längsachse (X) gewindebeweglich mit einem komplementären Gegengewinde (217) verschraubt ist.

8. Implantat (300) nach Anspruch 1 oder einem der Ansprüche 3 bis 6, **dadurch gekennzeichnet, dass** der Mechanismus (303) eine entlang der Längsachse (X) orientierte Drehachse (G) und ein um die Drehachse (G) beweglich gelagertes Exzenterelement (308) mit einer in Bezug auf die Drehachse (G) exzentrischen Kontur (311) aufweist, wobei unterschiedliche Abschnitte der Kontur (311) den ersten Abschnitt (301) und den zweiten Abschnitt (302) bilden, und wobei eine Drehung des Exzenterelements (308) ein Verspannen der Kontur (311) zwischen dem Außenumfang (DA) des Knochendeckels (D) und dem Innenumfang (KI) des Schädelknochens (K) bewirkt.

9. Implantat (300) nach Anspruch 8, **dadurch gekennzeichnet, dass** ein Schaftelement (307) vorhanden ist, an welchem einends das Exzenterelement (308) und andernends ein Werkzeugaufnahmeelement (319) angeordnet sind, wobei das Werkzeugaufnahmeelement (319) zum Aufbringen eines um die Drehachse (G) gerichteten Drehmoments eingerichtet ist, insbesondere wobei das Werkzeugaufnahmeelement (319) ein in Bezug auf die Längsachse (X) oberes Stirnende des Implantats (300) bildet und über einen Sollbruchabschnitt (317) mit dem Schaftelement (307) verbunden ist.

10. Implantat (300) nach einem der Ansprüche 8 oder 9, **dadurch gekennzeichnet, dass** das Exzenterelement (308) wenigstens einen ersten Klingenabschnitt (309), welches den ersten Abschnitt (301) bildet, und einen um die Drehachse (G) versetzt angeordneten zweiten Klingenabschnitt (310), welcher den zweiten Abschnitt (302) bildet, aufweist, wobei die Klingenabschnitte (309, 310) jeweils radial von der Drehachse (G) längserstreckt abragen und entgegengesetzt orientierte Schneiden (312, 313) aufweisen.

11. Implantat (500) nach einem der Ansprüche 1 bis 6, **dadurch gekennzeichnet, dass** der Mechanismus (503) ein erstes Zylinderelement (508) und ein zweites Zylinderelement (509) aufweist, deren Längsachsen (L1, L2) koaxial und jeweils parallel zu der Querachse (Y) ausgerichtet sind, wobei das erste Zylinderelement (508) eine profilierte erste Zylindermantelfläche (510) und eine den ersten Abschnitt (501) bildende erste Fläche (511) aufweist, wobei das zweite Zylinderelement (509) eine komplementär profilierte zweite Zylindermantelfläche (512) und eine den zweiten Abschnitt (502) bildende zweite Fläche (513) aufweist, wobei die erste Zylindermantelfläche (510) und die zweite Zylindermantelfläche (512) unter Ausbildung einer entlang der Querachse (Y) form- und/oder kraftschlüssigen Verbindung (V) in unterschiedlichen axialen Relativpositionen der Zylinderelemente (508, 509) miteinander verbindbar sind.

12. Implantat (500) nach Anspruch 11, **dadurch gekennzeichnet, dass** die zweite Fläche (513) eine Stirnendfläche des zweiten Zylinderelements (509) und zur radialen Anlage an dem Innenumfang (KI) des Schädelknochens (K) eingerichtet ist, und dass die erste Fläche (511) eine Außenmantelfläche des ersten Zylinderelements (508) und zum Einfügen in eine radial in den Außenumfang (DA) des Knochendeckels (D) erstreckte Bohrung (H) eingerichtet ist, insbesondere wobei die erste Zylindermantelfläche (510) eine Innenmantelfläche ist, und dass die zweite Zylindermantelfläche (512) eine Außenmantelfläche ist.

13. Implantat (500) nach Anspruch 11 oder 12, **dadurch gekennzeichnet, dass** die erste Zylindermantelfläche (510) und die zweite Zylindermantelfläche (512) jeweils eine Profilierung in Form einer Rastgeometrie (R1, R2) aufweisen und entlang der Querachse (Y) einseitig überrastbar miteinander verrastet sind.

14. Implantat (500) nach einem der Ansprüche 11 bis 13, **dadurch gekennzeichnet, dass** die erste Zylindermantelfläche (510) und die zweite Zylindermantelfläche (512) jeweils eine Profilierung in Form einer Gewindegeometrie (R1a, R2a) aufweisen und entlang der Querachse (Y) gewindebeweglich miteinander verschraubt sind.

15. Implantatsystem, aufweisend wenigstens zwei Implantate (100, 200, 300, 400, 500) nach einem der vorhergehenden Ansprüche.

## Claims

1. Implant (100, 200, 300, 400, 500) for fixing a cranial bone flap (D) in a cranial opening (B), comprising
a longitudinal axis (X), which is aligned along an axial direction (A) of the cranial opening (B),
a transverse axis (Y), which is aligned along a radial direction (R) of the cranial opening (B),
a first portion (101, 201, 301, 401, 501), which is designed to transfer force to the bone flap (D),
a second portion (102, 202, 302, 402, 502), which is designed to transfer force to a skull bone (K) encircling the cranial opening (B),
wherein the first portion (101, 201, 301, 401, 501) and/or the second portion (102, 202, 302, 402, 502) is designed for arrangement in an annular gap (S) formed between an outer circumference (DA) of the bone flap (D) and an inner circumference (KI) of the skull bone (K),
and comprising a mechanism (103, 203, 303, 403, 503), which is operatively connected to the first portion (101, 201, 301, 401, 501) and the second portion (102, 202, 302, 402, 502) and by means of which a transverse spacing (Q), projected onto the transverse axis (Y), between the first portion (101, 201, 301, 401, 501) and the second portion (102, 202, 302, 402, 502) can at least be enlarged, as a result of which the first portion (101, 201, 301, 401, 501) can be pressed radially (R) inwardly against the bone flap (D) and the second portion (102, 202, 302, 402, 502) can be pressed radially (R) outwardly against the skull bone (K),
wherein a supporting portion (220, 320, 420) is present, which is arranged above the first portion (201, 301, 401) and the second portion (202, 302, 402) along a longitudinal axis (X) and has an underside (221, 321, 421), which is designed for supporting on an outer side (KF) of the skull bone (K) and an outer side (DF) of the bone flap (D),
**characterized in that** the supporting portion (220) is connected to the rest of the implant (200) by means of a detachable joining connection, in particular a bayonet closure.

2. Implant (100, 200, 400, 500) as claimed in claim 1, **characterized in that** the mechanism (103, 203, 403, 503) is designed for translational displacement of the first portion (101, 201, 401, 501) and/or of the second portion (102, 202, 402, 502) along the transverse axis (Y).

3. Implant (300) as claimed in claim 1 or 2, **characterized in that** the mechanism (303) is designed for rotational displacement of the first portion (301) and/or of the second portion (302) about the longitudinal axis (X).

4. Implant (100, 200, 300, 400, 500) as claimed in one of the preceding claims, **characterized in that** the first portion (101, 201, 301, 401, 501) has a first contact surface, which is designed to radially bear against the outer circumference (DA) of the bone flap (D), and/or **in that** the second portion (102, 202, 302, 402, 502) has a second contact surface, which is designed to radially bear against the inner circumference (KI) of the skull bone.

5. Implant (100, 500) as claimed in one of the preceding claims, **characterized in that** the first portion (101, 501) has a joining surface (106, 511), which is designed for insertion into a bore (H) extending radially in the outer circumference (DA) of the bone flap (D).

6. Implant (200, 300, 400) as claimed in one of the preceding claims, **characterized in that** at least one mandrel portion (223, 323, 423) is present, which is elongate along the transverse axis (Y) and at one end has a mandrel tip (224, 324, 424) which projects beyond the first portion (201, 301, 401) and is designed to radially pierce into the outer circumference (DA) of the bone flap (D).

7. Implant (200) as claimed in one of the preceding claims, **characterized in that** the mechanism (203) has at least one clamping element (208) and a cone element (207), wherein the clamping element (208) is movably mounted along the transverse axis (Y) and has an inner cone surface (210) and an end face (211) forming the first portion (201) or the second portion (202), wherein the cone element (207) is movably mounted along the longitudinal axis (X) and has an outer cone surface (214) interacting with the inner cone surface (210), and wherein the clamping element (208) can be displaced by means of a movement of the cone element (207) along the transverse axis (Y), in particular wherein the mechanism (203) has a further clamping element (209), which can be oppositely displaced by means of the movement of the cone element (207) along the transverse axis (Y), and/or wherein the cone element (207) has a thread (216), which is screwed to a complementary mating thread (217) for threaded movement along the longitudinal axis (X).

8. Implant (300) as claimed in claim 1 or in one of claims 3 to 6, **characterized in that** the mechanism (303) has an axis of rotation (G), which is oriented along the longitudinal axis (X), and an eccentric element (308), which is movably mounted about the axis of rotation (G) and has a contour (311) which is eccentric with respect to the axis of rotation (G), wherein different portions of the contour (311) form the first portion (301) and the second portion (302), and wherein a rotation of the eccentric element (308) causes the contour (311) to be clamped between the outer circumference (DA) of the bone flap (D) and the inner circumference (KI) of the skull bone (K).

9. Implant (300) as claimed in claim 8, **characterized in that** a shaft element (307) is present, at one end of which the eccentric element (308) is arranged and at the other end of which a tool fitting element (319) is arranged, wherein the tool fitting element (319) is designed to apply a torque directed about the axis of rotation (G), in particular wherein the tool fitting element (319) forms an upper face end, with respect to the longitudinal axis (X), of the implant (300) and is connected to the shaft element (307) via a predetermined breaking portion (317).

10. Implant (300) as claimed in one of claims 8 or 9, **characterized in that** the eccentric element (308) has at least a first blade portion (309), which forms the first portion (301), and a second blade portion (310), which is offset about the axis of rotation (G) and forms the second portion (302), wherein the blade portions (309, 310) each project radially from the axis of rotation (G) with a longitudinal extent and have oppositely oriented cutting edges (312, 313).

11. Implant (500) as claimed in one of claims 1 to 6, **characterized in that** the mechanism (503) has a first cylinder element (508) and a second cylinder element (509), the longitudinal axes (L1, L2) of which are coaxial and are aligned parallel to the transverse axis (Y), wherein the first cylinder element (508) has a profiled first cylinder lateral surface (510) and a first surface (511) forming the first portion (501), wherein the second cylinder element (509) has a second cylinder lateral surface (512) with a complementary profile and a second surface (513) which forms the second portion (502), wherein the first cylinder lateral surface (510) and the second cylinder lateral surface (512) can be connected to one another to form a connection (V), which is form fitting and/or force fitting along the transverse axis (Y), in different axial relative positions of the cylinder elements (508, 509).

12. Implant (500) as claimed in claim 11, **characterized in that** the second surface (513) is a face end surface of the second cylinder element (509) and is designed to radially bear against the inner circumference (KI) of the skull bone (K), and **in that** the first surface (511) is an outer lateral surface of the first cylinder element (508) and is designed for insertion into a bore (H) which extends radially in the outer circumference (DA) of the bone flap (D), in particular wherein the first cylinder lateral surface (510) is an inner lateral surface, and **in that** the second cylinder lateral surface (512) is an outer lateral surface.

13. Implant (500) as claimed in claim 11 or 12, **characterized in that** the first cylinder lateral surface (510) and the second cylinder lateral surface (512) have a respective profiling in the form of a latching geometry (R1, R2) and are latched to each other along the transverse axis (Y) such that one can be latched over the other on one side.

14. Implant (500) as claimed in one of claims 11 to 13, **characterized in that** the first cylinder lateral surface (510) and the second cylinder lateral surface (512) have a respective profiling in the form of a threaded geometry (R1a, R2a) and are screwed to each other for threaded movement along the transverse axis (Y).

15. Implant system, comprising at least two implants (100, 200, 300, 400, 500) as claimed in one of the preceding claims.

## Revendications

1. Implant (100, 200, 300, 400, 500) destiné à fixer un volet crânien (D) dans une ouverture crânienne (B), comportant
un axe longitudinal (X), qui est orienté le long d'une direction axiale (A) de l'ouverture crânienne (B), un axe transversal (Y), qui est orienté le long d'une direction radiale (R) de l'ouverture crânienne (B),
une première section (101, 201, 301, 401, 501), qui est mise au point pour transférer une force sur le volet crânien (D),
une seconde section (102, 202, 302, 402, 502), qui est mise au point pour transférer une force sur un os crânien (K) bordant l'ouverture crânienne (B),
la première section (101, 201, 301, 401, 501) et/ou la seconde ouverture (102, 202, 302, 402, 502) étant mises au point pour être disposées dans une fente annulaire (S) formée entre une périphérie extérieure (DA) du volet crânien (D) et une périphérie intérieure (KI) de l'os crânien (K),
et comportant un mécanisme (103, 203, 303, 403, 503) relié en liaison fonctionnelle avec la première section (101, 201, 301, 401, 501) et la seconde section (102, 202, 302, 402, 502), au moyen duquel une distance transversale (Q) projetée sur l'axe transversal (Y) peut être au moins agrandie entre la première section (101, 201, 301, 401, 501) et la seconde section (102, 202, 302, 402, 502), ce qui permet de presser la première section (101, 201, 301, 401, 501) dans la direction radiale (R) vers l'intérieur sur le volet crânien (D) et la seconde section (102, 202, 302, 402, 502) dans la direction radiale (R) vers l'extérieur de l'os crânien (K),
une section d'appui (220, 320, 420) étant présente, laquelle est disposée le long de l'axe longitudinal (X) au-dessus de la première section (201, 301, 401) et de la seconde section (202, 302, 402) et qui comporte un côté inférieur (221, 321, 421), qui est mis au point pour être soutenu sur un côté extérieur (KF) de l'os crânien (K) et sur un côté extérieur (DF) du volet crânien (D),
**caractérisé en ce que** la section d'appui (220) est reliée par ailleurs à l'implant (200) au moyen d'une liaison d'assemblage amovible, en particulier d'une fermeture à baïonnette.

2. Implant (100, 200, 400, 500) selon la revendication 1, **caractérisé en ce que** le mécanisme (103, 203, 403, 503) est mis au point pour déplacer par translation la première section (101, 201, 401, 501) et/ou la seconde section (102, 202, 402, 502) le long de l'axe transversal (Y).

3. Implant (300) selon la revendication 1 ou 2, **caractérisé en ce que** le mécanisme (303) est mis au point pour déplacer en rotation la première section (301) et/ou la seconde section (302) autour de l'axe longitudinal (X).

4. Implant (100, 200, 300, 400, 500) selon l'une des revendications précédentes, **caractérisé en ce que** la première section (101, 201, 301, 401, 501) comporte une première surface de contact, laquelle est mise au point pour venir en appui radialement sur la périphérie extérieure (DA) du volet crânien (D), et/ou que la seconde section (102, 202, 302, 402, 502) comporte une seconde surface de contact, laquelle est mise au point pour venir en appui radialement sur la périphérie intérieure (KI) de l'os crânien.

5. Implant (100, 500) selon l'une des revendications précédentes, **caractérisé en ce que** la première section (101, 501) comporte une surface d'assemblage (106, 511), laquelle est mise au point pour être insérée dans un alésage (H) s'étendant radialement dans la périphérie extérieure (DA) du volet crânien (D).

6. Implant (200, 300, 400) selon l'une des revendications précédentes, **caractérisé en ce qu'**au moins une section de mandrin (223, 323, 423) est présente, laquelle est étirée en longueur le long de l'axe transversal (Y) et comporte du côté d'une extrémité une pointe (224, 324, 424) de mandrin dépassant au-delà de la première section (201, 301, 401), laquelle est mise au point pour être enfoncée dans la périphérie extérieure (DA) du volet crânien (D).

7. Implant (200) selon l'une des revendications précédentes, **caractérisé en ce que** le mécanisme (203) comporte au moins un élément de serrage (208) et un élément conique (207), l'élément de serrage (208) étant monté de manière mobile le long de l'axe transversal (Y) et comportant une surface de cône intérieur (210) ainsi qu'une surface frontale (211) formant la première section (201) ou la seconde section (202), l'élément conique (207) étant monté de manière mobile le long de l'axe longitudinal (X) et comportant une surface de cône extérieur (214) coopérant avec la surface de cône intérieur (210), et l'élément de serrage (208) pouvant être déplacé le long de l'axe transversal (Y) au moyen d'un déplacement de l'élément conique (207), en particulier le mécanisme (203) comportant un autre élément de serrage (209), lequel peut être déplacé à l'opposé le long de l'axe transversal (Y) au moyen du déplacement de l'élément conique (207), et/ou l'élément conique (207) comportant un filetage (216), lequel est vissé de manière mobile par un filetage à un contre-filetage (217) complémentaire le long de l'axe longitudinal (X).

8. Implant (300) selon la revendication 1 ou selon l'une des revendications 3 à 6, **caractérisé en ce que** le mécanisme (303) comporte un axe de rotation (G) orienté le long de l'axe longitudinal (X) et un élément excentrique (308) monté de manière mobile autour de l'axe de rotation (G), avec un contour (311) excentrique par rapport à l'axe de rotation (G), différentes sections du contour (311) formant la première section (301) et la seconde section (302), et une rotation de l'élément excentrique (308) entraînant un assemblage par serrage du contour (311) entre la périphérie extérieure (DA) du volet crânien (D) et la périphérie intérieure (KI) de l'os crânien (K).

9. Implant (300) selon la revendication 8, **caractérisé en ce qu'**un élément de tige (307) est présent, sur lequel l'élément excentrique (308) est disposé sur un côté et un élément de réception (319) d'outil est disposé sur l'autre côté, l'élément de réception (319) d'outil étant mis au point pour appliquer un couple de rotation dirigé autour de l'axe de rotation (G), en particulier l'élément de réception (319) d'outil formant une extrémité frontale supérieure de l'implant (300) par rapport à l'axe longitudinal (X) et étant relié à l'élément de tige (307) par une section de rupture théorique (317).

10. Implant (300) selon l'une des revendications 8 ou 9, **caractérisé en ce que** l'élément excentrique (308) comporte au moins une première section de lame (309), laquelle forme la première section (301), et une seconde section de lame (310) disposée avec un décalage autour de l'axe de rotation (G), laquelle forme la seconde section (302), les sections de lame (309, 310) dépassant chacune radialement de l'axe de rotation (G) avec une extension longitudinale et comportant des tranchants (312, 313) orientés à l'opposé.

11. Implant (500) selon l'une des revendications 1 à 6, **caractérisé en ce que** le mécanisme (503) comporte un premier élément de cylindre (508) et un second élément de cylindre (509), dont les axes longitudinaux (L1, L2) sont orientés coaxialement et respectivement parallèlement à l'axe transversal (Y), le premier élément de cylindre (508) comportant une première surface d'enveloppe (510) de cylindre profilée et une première surface (511) formant la première section (501), le second élément de cylindre (509) comportant une seconde surface d'enveloppe (512) de cylindre profilée de manière complémentaire et une seconde surface (513) formant la seconde section (502), la première surface d'enveloppe (510) de cylindre et la seconde surface d'enveloppe (512) de cylindre pouvant être reliées l'une à l'autre en réalisant une liaison (V) par complémentarité de forme et/ou à force le long de l'axe transversal (Y) dans différentes positions relatives axiales des éléments de cylindre (508, 509).

12. Implant (500) selon la revendication 11, **caractérisé en ce que** la seconde surface (513) est une surface d'extrémité frontale du second élément de cylindre (509) et est mise au point pour venir en appui radialement sur la périphérie intérieure (KI) de l'os crânien (K), et que la première surface (511) est une surface d'enveloppe extérieure du premier élément de cylindre (508) et est mise au point pour être insérée dans un alésage (H) s'étendant radialement dans la périphérie extérieure (DA) du volet crânien (D), en particulier la première surface d'enveloppe (510) de cylindre étant une surface d'enveloppe intérieure, et que la seconde surface d'enveloppe (512) de cylindre est une surface d'enveloppe extérieure.

13. Implant (500) selon la revendication 11 ou 12, **caractérisé en ce que** la première surface d'enveloppe (510) de cylindre et la seconde surface d'enveloppe (512) de cylindre comportent chacune un profilage sous la forme d'une géométrie d'enclenchement (R1, R2) et sont enclenchées l'une avec l'autre de manière à pouvoir se chevaucher par enclenchement sur un côté le long de l'axe transversal (Y).

14. Implant (500) selon l'une des revendications 11 à 13, **caractérisé en ce que** la première surface d'enveloppe (510) de cylindre et la seconde (512) de cylindre comportent chacune un profilage sous la forme d'une géométrie filetée (R1a, R2a) et sont vissées l'une à l'autre de manière mobile par un filetage le long de l'axe transversal (Y).

15. Système d'implants comportant au moins deux implants (100, 200, 300, 400, 500) selon l'une des revendications précédentes.
